(19) Europäisches Patentamt
European Patent Office
Office européen des brevets

(11) EP 1 202 993 B1

(12) **FASCICULE DE BREVET EUROPEEN**

(45) Date de publication et mention de la délivrance du brevet: **19.10.2005 Bulletin 2005/42**

(21) Numéro de dépôt: **00958679.3**

(22) Date de dépôt: **11.08.2000**

(51) Int Cl.[7]: **C07D 471/16**, A61K 31/4375, A61P 35/00 // (C07D471/16, 221:00, 221:00), C07D221:00

(86) Numéro de dépôt international: **PCT/FR2000/002313**

(87) Numéro de publication internationale: **WO 2001/012632 (22.02.2001 Gazette 2001/08)**

# (54) DERIVES DE PHENANTHROLINE-7-ONES ET LEURS APPLICATIONS EN THERAPEUTIQUE

PHENANTHROLIN-7-ONDERIVATE UND IHRE THERAPEUTISCHE VERWENDUNG

PHENANTHROLINE-7-ONE DERIVATIVES AND THEIR THERAPEUTIC USES

(84) Etats contractants désignés: **AT BE CH CY DE DK ES FI FR GB GR IE IT LI LU MC NL PT SE**

(30) Priorité: **13.08.1999 FR 9910493**

(43) Date de publication de la demande: **08.05.2002 Bulletin 2002/19**

(73) Titulaire: **LABORATOIRE L. LAFON**
**94701 Maisons Alfort (FR)**

(72) Inventeurs:
- **DELFOURNE, Evelyne**
  **F-66450 Pollestres (FR)**
- **DARRO, Francis**
  **B-1070 Bruxelles (BE)**
- **BASTIDE, Jean**
  **F-66000 Perpignan (FR)**
- **KISS, Robert**
  **B-1440 Wauthier-Braine (BE)**
- **FRYDMAN, Armand**
  **F-91370 Verrières-le-Buisson (FR)**

(74) Mandataire: **Bernasconi, Jean Raymond et al**
**c/o Cabinet Lavoix,**
**2, Place d'Estienne d'Orves**
**75441 Paris Cédex (FR)**

(56) Documents cités:
**US-A- 5 182 287**

- **MATSUMOTO, SANDRA S. ET AL: "Mechanism of action studies of cytotoxic marine alkaloids: ascididemin exhibits thiol-dependent oxidative DNA cleavage" TETRAHEDRON LETT. (2000), 41(10), 1667-1670 , XP004189818**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 202 993 B1

## Description

La présente invention concerne des compositions pharmaceutiques à base de composés polyaromatiques utiles notamment comme médicaments antitumoraux.

En 1999, les traitements cytotoxiques (chimiothérapie) utilisés pour réduire la taille des tumeurs cancéreuses, contenir le développement du processus tumoral voire, dans trop peu de cas encore, supprimer les amas de cellules cancéreuses et le risque de métastases, combinent des substances chimiques d'introduction récente avec d'autres qui sont utilisées depuis quelques dizaines d'années. Par exemple, au 5-fluorouracil (5-FU), reconnu depuis près de 40 ans comme l'un des traitements les plus actifs du cancer colo-rectal, peut être substitué l'un ou l'autre des inhibiteurs spécifiques de la topoisomérase I (irinotécan ou topotécan) lorsque la tumeur n'est plus sensible au 5-FU. Plus généralement, l'arsenal thérapeutique disponible pour traiter les tumeurs colo-rectales va également s'enrichir avec la mise à disposition de l'oxaliplatine, des nouveaux "donneurs" in situ de 5-FU ou des inhibiteurs sélectifs de la thymidylate synthétase. Cette co-existence ne se limite pas au traitement des cancers colo-rectaux puisque, également, la chimiothérapie des cancers du sein, de l'ovaire, du poumon fait maintenant largement appel à la famille des dérivés des taxanes (paclitaxel, docetaxel). Le besoin de traitements plus efficaces et mieux tolérés, améliorant ainsi la survie et la qualité de vie des malades est impérieux puisque, en prenant toujours l'exemple des tumeurs colo-rectales, il a été estimé (S.L. Parker, T. Tong, S. Bolden *et al*., CA Cancer J. Clin., 1997) que, rien qu'aux Etats-Unis plus de 131 000 nouveaux cas ont été diagnostiqués en 1997, dont 54 000 étaient responsables du décès des patients. C'est la connaissance de cette situation qui a incité les inventeurs à s'intéresser à une famille de composés polyaromatiques encore peu étudiés, identifiés chez des Ascidies de mers chaudes, pour développer une chimie médicinale originale destinée à sélectionner des composés synthétiques issus d'un travail de conception/modulation chimique et doués d'une activité cytotoxique significative au plan thérapeutique.

Les mers et les océans qui couvrent plus de 70 % de la surface du globe, hébergent des plantes marines et des éponges dont l'étude pharmacognosique systématique progressive montre que ces espèces vivantes peuvent contenir des alcaloïdes complexes présentant des propriétés pharmacologiques intéressantes. Par exemple, les éponges *Cryptotheca crypta* et *Halichondria okadai* font l'objet d'études approfondies depuis la découverte de la présence, dans leurs cellules, de cytarabine ou d'balichondrine B. Il en est de même pour la famille des tuniciers, depuis l'isolement de l'aplidine du tunicier *Aplidium albicans* qui vit dans les îles Baléares (Espagne). Des alcaloïdes à structure tétrahydroisoquinolone ont été isolés de l'ascidie *Ecteinascidia turbinata*. Parmi ceux-ci, l'ecteinascidin-743 fait l'objet de travaux pré-cliniques approfondis (E. Igbicka *et al*., NCI-EORTC symposium, 1998; Abst. 130 p.34), ainsi que d'essais cliniques destinés à définir son potentiel thérapeutique comme médicament anticancéreux (A. Bowman *et al*., NCI-EORTC symposium, 1998; Abst. 452 p.118 ; M.Villanova-Calero e*t al.,* NCI-EORTC symposium, 1998; Abst. 453 p.118 ; M.J.X. Hillebrand *et al*., NCI-EORTC symposium, 1998; Abst. 455 p.119; E. Citkovic *et al*., NCI-EORTC symposium, 1998; Abst. 456 p.119). De nouveaux dérivés d'acridines pentacycliques font également l'objet de travaux de pharmaco-chimie (D.J. Hagan *et al*., J. Chem. Soc., Perkin Transf., 1997; 1: 2739-2746).

Autre alcaloïde naturel d'origine marine, l'ascididémine a été extraite du tunicier *Didemnum sp.* (J. Kobayashi *et al*., Tehahedron, lett. 1988 ; 29 : 1177-80) et de l'ascidie *Cystodytes dellechiajei* (I. Bonnard *et al,* Anti-cancer Drug design 1995 ; 10 : 333-46). L'ascididémine possède des propriétés antiprolifératives mises en évidence sur le modèle de leucémie murine (lignées P388 ou L1210) et décrites par F.J. Schmitz *et al*. (J. Org. Chem. 1991 ; 56 : 804-8), B. Lindsay *et al*. (Bioorg. Med. Chem. Lett. 1995 ; 5 : 739-42) et J. Kobayashi *et al*. (Tehahedron lett. 1988 ; 29 : 1177-80), et sur le modèle de leucémie humaine telles que décrites par I. Bonnard *et al*. (Anti-cancer Drug design 1995 ; 10 : 333-46). On peut également citer la 2-bromoleptoclinidone isolée de l'ascidie L*eptoclinides sp*. par S.J. Bloor *et al*. (J. Ann. Chem. Soc. 1987 ; 109 : 6134-6) et synthétisée par F. Bracher *et al*. (Hétérocycles 1989 ; 29 : 2093-95) puis par M.E. Jung *et al*. (Hétérocycles 1994 ; 39 ; 2 : 767-778). La 2-bromoleptoclinidone présente une cytoxicité sur le modèle cellulaire de leucémie avec une ED 50 de 0,4 μg/ml. Les propriétés cytotoxiques ont été confirmées, par F. Bracher (Pharmazie 1997 ; 52 : 57-60) aussi bien *in vitro* - sur soixante lignées cellulaires tumorales en culture - que *in vivo* sur les modèles de xénogreffes de lignées cellulaires tumorales humaines (tumeurs du colon SW-620 et HTC116, tumeur rénale A498 et mélanome LOX IM VI) implantées chez des souris.

D'autres composés dérivés de l'ascididémine tels que la 11-hydroxy ascididémine, la 11-méthoxy ascididémine, les 11-phényle et 11-nitrophényle ascididémines, les 1-nitro et 3-nitro ascididémines et la néocalliactine ont été décrits au plan chimique par différentes équipes telles que celles de F.J. Schmitz (J. Org. Chem. 1991 ; 56 : 804-8) et de Y. Kitahara *et al*. (Heterocycles 1993 ; 36 : 943-46 ; Tetrahedron Lett. 1997 ; 53, 17029-38), G. Gellerman *et al*. (Tetrahedron lett. 1993 ; 34 : 1827-30), S. Nakahara *et al* (Heterocycles 1993; 36 : 1139-44), I. Spector *et al*. (US -A 5 432 172).

La méridine, est un autre alcaloïde naturel extrait de l'ascidie *Amphicarpa meridiana* ou de l'éponge marine *Corticum sp.* La méridine a été isolée par F.J. Schmitz *et al*. (J. Org. Chem. 1991; 56: 804 - 808) puis décrite pour ses propriétés antiprolifératives sur modèle de leucémie murine (P388) et antifongiques dans le brevet US A5 182 287 (Gunawardana *et al*. du 23 Janvier 1993). Ses propriétés cytotoxiques sur deux lignées cellulaires humaines: cellules

de cancer du colon (HT-29) et carcinome du poumon (A549) ont été rapportées par R.E. Longley *et al*. (J. of Nat. Products 1993; 56: 915-920).

Parmi ces composés, on peut citer également la cystodamine, alcaloïde pentacyclique isolé de l'ascidie *Cystodytes dellechiajei* par N. Bontemps *et al*. (Tetrahedron lett., 1994; 35 : 7023-7026) qui présente une activité cytotoxique sur des lymphoblastes de leucémie humaine.

La présente invention a pour objet des composés de formule générale I et la

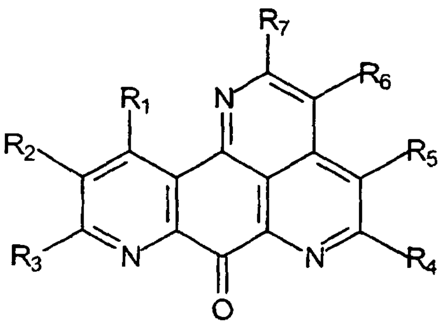

Formule I

et

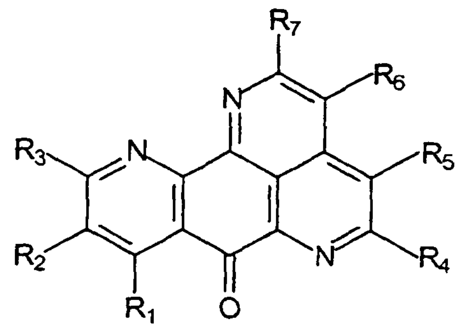

Formule Ia

dans lesquelles :

$R_1$, $R_2$, $R_3$, $R_4$ et $R_5$ sont choisis parmi l'hydrogène, les halogènes, les groupes alkyle en $C_1$-$C_6$, hydroxy, -CHO, $-OR_8$, -COOH, -CN, $-CO_2R_8$, $-CONHR_8$, $-CONR_8R_9$, -NH2, $-NHR_8$, $-N(R_8)_2$ $-NH-CH_2-CH_2-N(CH_3)_2$, $-NH-CH_2-CH_2-Cl$, $-NHCOR_8$, morpholino, nitro, $SO_3H$,

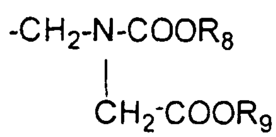

,

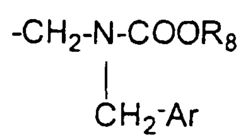

$R_8$ et $R_9$ étant choisis parmi les groupes alkyle en $C_1$-$C_6$, et les groupes phénylalkyle ($C_1$-$C_4$) et Ar étant un groupe aryle en $C_6$-$C_{14}$,

- $R_6$ est l'hydrogène,
- R7 est l'hydrogène,

et les sels d'addition de ces composés avec des acides pharmaceutiquement acceptables.

Un groupe particulier de composés de formule I et/ou la est ceux dans lesquels : $R_1$, $R_2$, $R_3$, $R_4$ et $R_5$ sont choisis parmi l'hydrogène, les halogènes, les groupes alkyle en $C_1$-$C_6$, hydroxy, -CHO, $-OR_8$, -COOH, -CN, $-CO_2R_8$, $-CONHR_8$, $-CONR_8R_9$, $-NH_2$, $-NHR_8$, $-N(R_8)_2$ $-NH-CH_2-CH_2-N(CH_3)_2$, $-NHCOR_8$, morpholino, nitro , $SO_3H$,

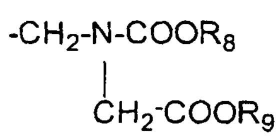

,

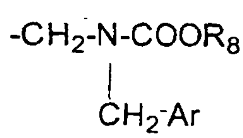

$R_8$ et $R_9$ étant choisis parmi les groupes alkyle en $C_1$-$C_6$, et Ar étant un groupe aryle en $C_6$-$C_{14}$,

La présente invention a plus particulièrement pour objet les composés choisis parmi les composés de formule (I) et de formule (la) dans lesquels $R_1$, $R_2$, $R_3$, $R_4$ et $R_5$ sont choisis parmi l'hydrogène, les halogènes, les groupes alkyle en $C_1$-$C_6$, hydroxy, $-OR_8$, $NO_2$, $-NH_2$, $-NHR_8$, $- NH(R_8)_2$, $-NH-CH_2-CH_2-N(CH_3)_2$, $-NH-CH_2-CH_2-Cl$, $-NHCOR_8$,

$R_8$ étant choisi parmi les groupes alkyle en $C_1$-$C_6$,
et les sels d'addition de ces composés avec des acides pharmaceutiquement acceptables.

Un groupe de composés préférés est celui constitué par les composés de formule I et Ia dans lesquels au moins l'un des groupes $R_1$, $R_2$, $R_3$, $R_4$ ou $R_5$ est un groupe $OR_8$.

Les "sels d'addition avec des acides pharmaceutiquement acceptables" désignent les sels qui donnent les propriétés biologiques des bases libres, sans avoir d'effet indésirable. Ces sels peuvent être notamment ceux formés avec des acides minéraux, tels que l'acide chlorhydrique, l'acide bromhydrique, l'acide sulfurique, l'acide nitrique, l'acide phosphorique; des sels métalliques acides, tels que l'orthophosphate disodique et le sulfate monopotassique, et des acides organiques.

De manière générale, les composés de formules I ou Ia dans lesquelles $R_6$ et $R_7$ sont des hydrogènes peuvent être obtenus par un procédé qui consiste à :

a) faire réagir :

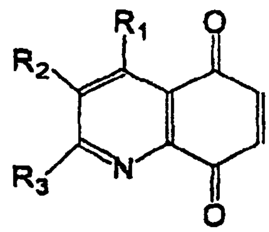

IV

et un azadiène de formule

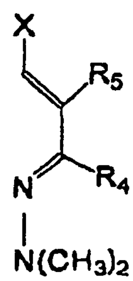

V

où X = $CH_2$-$CH_2$-NHBoc
pour obtenir un mélange de composés

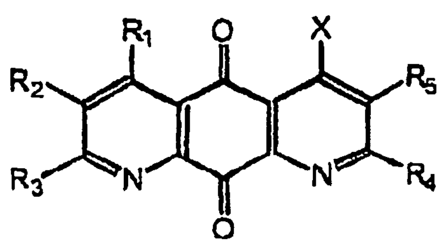

+

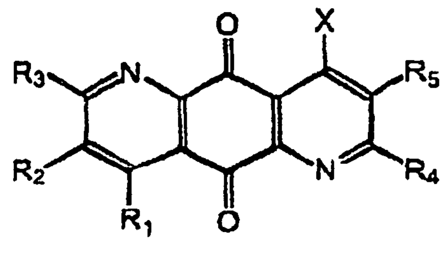

Formule II

Formule IIa

b) éventuellement séparer les composés de formules II et IIa,
c) cycliser un composé de formules II et/ou IIa pour obtenir un composé de formules I et/ou Ia,
d) éventuellement séparer les composés de formules I ou Ia.

La réaction de cyclisation des composés de formules III et IIIa peut être obtenue à chaud en présence de $NH_4Cl$ dans un solvant approprié.

Lorsque X = $CH_2$-$CH_2$-NHBoc
les composés de formules I et la sont obtenus directement en présence de $NaHCO_3$ en milieu acide trifluoroacétique à partir des composés de formules II et IIa.
Un exemple d'azadiène substitué peut-être préparé selon le schéma suivant :

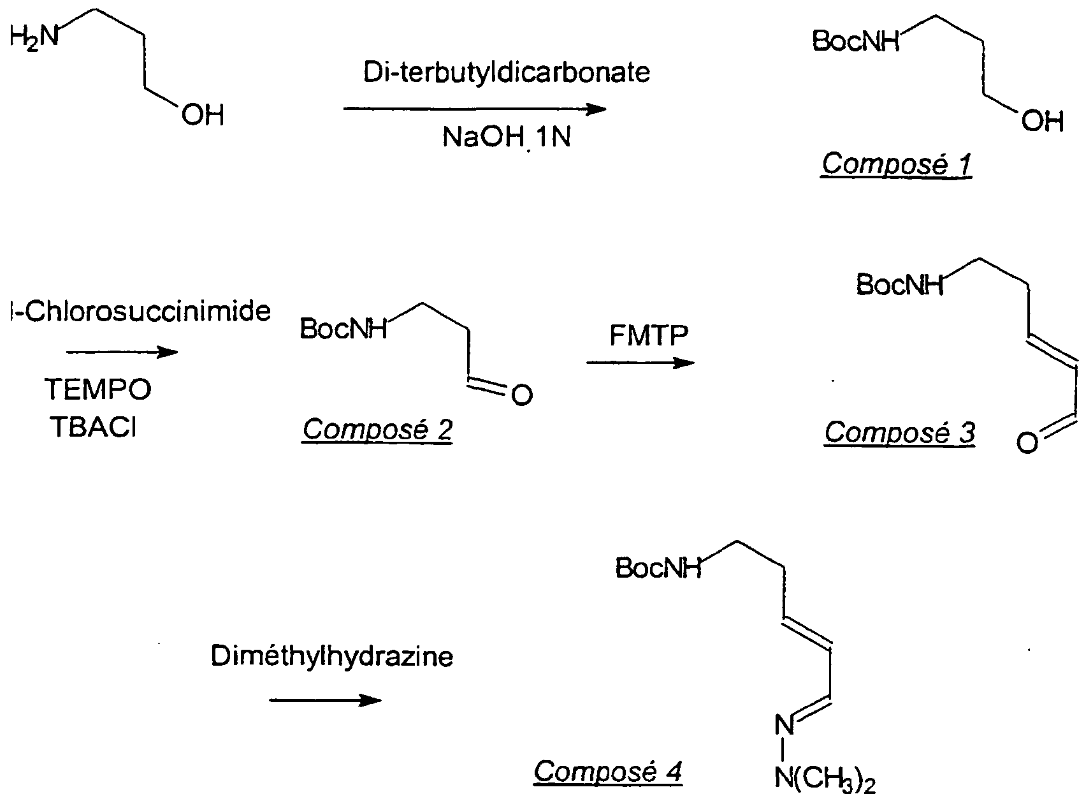


TEMPO = tétraméthyl-1-pipéridinyloxy, radical libre
TBACl = chlorure de tétrabutyl ammonium
FMTP = formylméthylènetriphénylphosphorane

Les exemples suivants illustrent la préparation des composés de formules (I) et (la).

**A - Préparation de l'azadiène (composé 4)**

**A-1 - Synthèse du N-BOC-1-amino-2-hydroxy-propane (Composé 1)**

A une solution de 2ml (27 mmol) de 3-amino-1-propanol dans un mélange de 60 ml de dioxanne, 30 ml d'eau et 30 ml de NaOH 1N, on ajoute à 0 °C, 4,2 g (29,7 mmol) de di-*tert*-butyldicarbonate. Le milieu réactionnel est maintenu sous agitation à température ambiante une nuit puis il est acidifié à pH 1 à l'aide d'HCl concentré. Après plusieurs extractions (3 fois 50 ml) par l'acétate d'éthyle (AcOEt), les phases organiques sont séchées sur $MgSO_4$ puis concentrées à l'évaporateur rotatif pour donner 4 g de produit attendu sous forme d'une huile jaune:

- Rendement : 85 %.
- $^1$H RMN ($CDCl_3$) : 1,25 (s, 9H) ; 2,50 (m, 2H) ; 3,05 (m, 2H) ; 3,45 (m, 2H) ; 5,40 (s large, 1H).

**A-2 - Synthèse du N-BOC-3-amino-propanal (Composé 2)**

18 g (103 mmol) de composé 1, 1,62 g (10,4 mmol) de TEMPO (tétraméthyl-1-pipéridinyloxy, radical libre), 2,9 g (10,45 mmol) de chlorure de tétrabutyl ammonium et 21 g (75,5 mmol) de N-chlorosuccinimide sont mis en suspension dans 351 ml de $NaHCO_3$ / $K_2CO_3$ (0,5 N / 0,05 N) et 351 ml de $HCCl_3$. Le milieu réactionnel est fortement agité pendant 2 heures. La phase organique est décantée, séchée sur $MgSO_4$ puis concentrée à l'évaporateur rotatif pour donner l'aldéhyde attendue sous forme d'huile orange clair.

- Rendement : 100 %.
- $^1H$ RMN ($CDCl_3$) : 1,35 (s, 9H) ; 2,44 (d, 2H, J = 6,8 Hz) ; 3,21 (m, 2H) ; 4,90 (s large, 1 H) ; 6,04 (dd, 1H, J = 8 et 15,6 Hz) ; 6,74 (td, 1H, J = 6,8 et 15,6 Hz) ; 9,39 (d, 1H, J = 8 Hz).

**A-3 - Synthèse du N-BOC-5-amino-2-penten-1-al (Composé 3)**

11 g (66,7 mmol) de composé 2 et 24,3 g (80 mmol) de formylméthylènetriphénylphosphorane (FMTP) sont solubilisés dans 350 ml de benzène puis le milieu réactionnel est porté à reflux pendant 9 heures. Après évaporation du solvant à l'évaporateur rotatif, le résidu est filtré une première fois sur silice [($CHCl_3$ /heptane 1 : 1) puis $CHCl_3$] pour éliminer la triphénylphosphine. Une deuxième filtration sur silice (AcOEt / heptane 8 : 2) permet d'obtenir 3,88 g de composé 3 sous forme d'une huile jaune-orange.

- Rendement : 29 %.
- $^1H$ RMN ($CDCl_3$) : 1,47 (s, 9H) ; 2,60 (m, 2H) ; 3,38 (m, 2H), 4,82 (s large, 1H) ; 6,18 (dd, 1H) ; 6,88 (td, 1H) ; 9,55 (d, 1H).

**A-4 - Synthèse de la diméthylhydrazone du N-BOC-5-amino-2-penten-1-al (Composé 4)**

A 1,47 ml (19,5 mmol) de diméthylhydrazine et 8 gouttes d'acide acétique dans 30 ml d'éther sont ajoutés à 0 °C 3,88 g (19,5 mmol) de composé 3. Le milieu réactionnel est laissé sous agitation 10 min, la phase organique est décantée, lavée par de l' HCl 1 N puis par une solution saturée de NaCl. Après séchage sur $MgSO_4$ et évaporation du solvant à l'évaporateur rotatif, 4,4 g d'hydrazone (composé 4) sont obtenus sous forme d'huile jaune-orangé.

- Rendement : 94 %.
- $^1H$ RMN ($CDCl_3$) : 2,30 (s, 9H) ; 2,3 (m, 2H) ; 2,82 (m, 2H), 4,52 (s large, 1H) ; 5,70 (td, 1H, J = 6,8 et 15,6 Hz) ; 6,22 (ddd, 1H, J = 0,8 et 8,8 et 15,6 Hz) ; 6,96 (d, 1H, J = 8,8 Hz).
- $^{13}C$ RMN ($CDCl_3$) : 28,15 ; 33,05 ; 39,58 ; 42,51 ; 78,77 ; 130,84 ; 130,95 ; 135,54 ; 155,68.

**B - Préparation des composés de formule II et IIa**

**B-1 : Synthèse de la 4-méthylpyrido[2,3-g]quinoline-5,10-dione (intermédiaire I-1b) et de la 4-méthylpyrido [3,2-g]quinoline-5,10-dione (intermédiaire II-1b)**

Un mélange de 0,5 g (3,14 mmol) de quinoline-5,8-dione, 0,35 g (3,14 mmol) de diméthylhydrazone du crotonaldéhyde et 0,45 ml (4,76 mmol) d'anhydride acétique dans 20 ml de $CHCl_3$ sont traités dans un bain à ultrasons pendant 1 heure. Après évaporation du solvant à l'évaporateur rotatif, le milieu réactionnel est filtré sur silice ($CHCl_3$) pour donner 0,428 g de mélange des deux isomères I-1a et II-1a sous forme de poudre violette. Cette poudre et 1,6 g (18,4 mmol) de $MnO_2$ sont mis en suspension dans 20 ml de $CHCl_3$ et le mélange est porté à reflux pendant 2 heures. Après filtration sur célite, le filtrat est concentré à l'évaporateur rotatif puis purifié par flash-chromatographie sur colonne de silice ($CH_2Cl_2$/MeOH 98 : 2) pour donner :

**Intermédaire (I-1b) : la 4-méthylpyrido[2,3-g]quinoline-5,10-dione :**

- 40 mg (Rendement : 6 %) sous forme de poudre marron.
- Point de fusion : 220 °C.
- $^1H$ RMN ($CDCl_3$) : 2,91 (s, 3H) ; 7,54 (d, 1H, J = 4,8 Hz) ; 7,75 (dd, 1H, J = 4 et 7,6 Hz) ; 8,67 (dd, 1H, J = 2 et 7,6 Hz) ; 8,91 (d, 1H, J = 4,8 Hz) ; 9,12 (dd, 1H, J = 2 et 4 Hz).
- $^{13}C$ RMN ($CDCl_3$) : 22,75 ; 127,93 ; 128,04 ; 129,32 ; 131,50 ; 135,50 ; 148,73 ; 149,26 ; 152,11 ; 153,68; 155,47 ;

181,46 ; 182,87.

- IR ($CHCl_3$) : 1689 $cm^{-1}$.

**Intermédiaire (II-1b) : la 4-méthylpyrido[3,2-g]quinoline-5,10-dione**

- 160 mg (Rendement : 23 %) sous forme d'une poudre marron.
- Point de fusion : 270 °C.
- $^1H$ RMN ($CDCl_3$) : 2,94 (s, 3H) ; 7,52 (d, 1H, J = 4,8 Hz) ; 7,76 (dd, 1H, J = 4,8 et 8,4 Hz) ; 8,59 (dd, 1H, J = 2 et 8,4 Hz) ; 8,92 (d, 1H, J = 4,8 Hz) ; 9,11 (dd, 1H, J = 2 et 4,8 Hz).
- $^{13}C$ RMN ($CDCl_3$): 22,81 ; 128,30 ; 128,39 ; 130,84 ; 131,55 ; 135,52 ; 147,90; 149,95 ; 151,74 ; 153,94 ; 155,35 ; 180,42 ; 184,02. IR ($HCCl_3$) 1672 ; 1700.

**B-2 : Synthèse de la 9-méthoxy-4-méthylpyrido[2,3-g]quinotine-5,10-dione (intermédiaire I-2b) et de la 6-méthoxy-4-méthylpyrido[3,2-g]quinoline-5,10-dione (intermédiaire II-2b)**

Un mélange de 0,5 g (2,8 mmol) de 4-méthoxy-quinoline-5,8-dione, 0,32 g (2,87 mmol) de diméthylhydrazone du crotonaldéhyde et 0,4 ml (4,23 mmol) d'anhydride acétique dans 8 ml de $CHCl_3$ sont portés à reflux pendant 1 heure. Après évaporation du solvant à l'évaporateur rotatif, le milieu réactionnel est filtré sur silice ($CH_2Cl_2$/MeOH 98 : 2) pour donner 0,48 g de mélange des deux isomères I-2a et II-2a sous forme de poudre violette. Cette poudre et 2,3 g (26,45 mmol) de $MnO_2$ sont mis en suspension dans 26 ml de $CHCl_3$ et le mélange est porté à reflux pendant 2 heures. Après filtration sur célite, le filtrat est concentré à l'évaporateur rotatif puis purifié par flash-chromatographie sur colonne de silice ($CH_2Cl_2$/MeOH 98 : 2) pour donner :

**Intermédiaire I-2b : la 9-méthoxy-4-méthylpyrido[2,3-g]quinoline-5,10-dione**

- 57 mg (Rendement : 8 %) sous forme de poudre rouge.
- $^1H$ RMN ($CDCl_3$) : 2,84 (s, 3H) ; 4,06 (s, 3H) ; 7,18(d, 1H, J = 6 Hz) ; 7,46 (d, 1H, J = 4,4 Hz) ; 8,87 (d, 1H, J = 6 Hz) ; 8,87 (d, 1H, J = 4,4 Hz).

**Intermédiaire II-2b : la 6-méthoxy-4-méthylpyrido[3,2-g]quinoline-5,10-dione**

- 293 mg (Rendement : 40 %) sous forme d'une poudre orange.
- $^1H$ RMN ($CDCl_3$) : 2,80 (s, 3H) ; 4,05 (s, 3H) ; 7,2 (d, 1H, J = 6 Hz) ; 7,48 (d, 1H, J = 4,8 Hz) ; 8,85 (d, 1H, J = 6 Hz) ; 8,88 (d, 1H, J = 4,8 Hz).
- $^{13}C$ RMN ($CDCl_3$) : 21,75 ; 43,41 ; 112,74 ; 119,72 ; 130,93 ; 131,04 ; 148,32 ; 149,22 ; 150,26 ; 151,60 ; 152,80 ; 155,11 ; 181,44 ; 184,53.
- IR ($CHCl_3$) : 1675 ; 1700 $cm^{-1}$.

**B-3 : Synthèse de la 9-nitro-4-méthylpyrido[2,3-g]quinoline-5,10-dione (intermédiaire I-5b) et de la 6-nitro-4-méthylpyrido[3,2-g]quinoline-5,10-dione (intermédiaire II-5b)**

Un mélange de 0,8 g (3,92 mmol) de 4-nitro-quinoline-5,8-dione, 0,65 g (5,8 mmol) de diméthylhydrazone du crotonaldéhyde et 0,55 ml (5,8 mmol) d'anhydride acétique dans 10,5 ml de $CHCl_3$ sont traités dans un bain à ultrasons 30 min. Après évaporation du solvant à l'évaporateur rotatif, le milieu réactionnel est filtré sur silice ($CH_2Cl_2$/MeOH 98 : 2) pour donner 0,7 g de mélange des deux isomères I-5a et II-5a sous forme de poudre violette. Cette poudre et 2,9 g (33,4 mmol) de $MnO_2$ sont mis en suspension dans 29 ml de $CHCl_3$ et le mélange est porté à reflux pendant 2 heures. Après filtration sur célite, le filtrat est concentré à l'évaporateur rotatif puis purifié par flash-chromatographie sur colonne de silice ($CH_2Cl_2$/MeOH 98 : 2) pour donner :

**Intermédiaire I-5b : la 9-nitro-4-méthylpyrido[2,3-g]quinoline-5,10-dione**

- 110 mg (Rendement : 11 %) sous forme de poudre.
- $^1H$ RMN ($CDCl_3$) : 2,98 (s, 3H) ; 7,19 (d, 1H, J = 5,6 Hz) ; 7,54 (d, 1H, J = 4,8 Hz) ; 8,79 (d, 1 H, J = 5,6 Hz) ; 8,94 (d, 1H, J = 4,8 Hz).
- IR ($HCCl_3$) : 1703 $cm^{-1}$.

**Intermédiaire II-5b : la 6-nitro-4-méthylpyrido[3,2-g]quinotine-5,10-dione**

- 165 mg (Rendement : 16 %) sous forme d'une poudre jaune-marron.
- $^1H$ RMN ($CDCl_3$): 2,85 (s, 3H) ; 7,6 (d, 1H, J = 4,8 Hz) ; 7,74 (d, 1H, J = 4,8 Hz) ; 8,99 (d, 1H, J = 4,8 Hz) ; 9,33 (d, 1H, J = 4,8 Hz).

**B-4 : Synthèse de la 9-diméthylamino-4-méthylpyrido[2,3-g]quinoline-5,10-dione (intermédiaire I-3b) et de la 6-diméthylamino-4-méthylpyrido[3,2-g]quinoline-5,10-dione (intermédiaire II-3b)**

150 mg (0,558 mmol) de tricycle nitré I-5a ou II-5a et 0,4 ml (1,95 mmol) de N,N-diméthylformamide diéthyl acétal sont solubilisés dans 2,1 ml de DMF et le milieu réactionnel est chauffé à 130 °C pendant 1 heure. Après évaporation du solvant à la pompe à vide, on obtient 140 mg de composé intermédiaire II-3a ou II-3b qui seront utilisés tels quels dans l'étape suivante:

**Intermédiaire II-3b : la 6-diméthylamino-4-méthylpyrido[3,2-g]quinoline-5,10 dione**

- Rendement : 94 %.
- $^1H$ RMN ($CDCl_3$) : 2,77 (s, 3H) ; 3,05 (s, 6H) ; 6,89 (d, 1H, J = 6 Hz) ; 7,39 (d, 1H, J = 4,8 Hz); 8,42 (d, 1H, J = 6 Hz) ; 8,74 (d, 1H, J = 4,8 Hz).

**B-5 : Synthèse de la 9-chloro-4-(N-BOC-1-aminoéthane)-5,10-dihydropyrido[2,3-g]quinoline-5,10-dione (intermédiaire I-7b) et de la 6-chloro-4-(N-BOC-1-aminoéthane)-5,10-dihydropyrido[3,2-g]quinoline-5,10-dione (intermédiaire II-7b)**

Un mélange de 0,6 g (3,1 mmol) de 4-chloro-quinoline-5,8-dione, 0,75 g (3,1 mmol) de diméthylhydrazone 4 et 0,45 ml (4,76 mmol) d'anhydride acétique dans 8,5 ml de $CHCl_3$ sont traités dans un bain à ultrasons pendant 30 min. Après évaporation du solvant à l'évaporateur rotatif, le milieu réactionnel est additionné de 2,7 g (31,1 mmol) de $MnO_2$ et de 22 ml de $CHCl_3$ et le mélange est porté à reflux pendant 2 heures. Après filtration sur célite, le filtrat est concentré à l'évaporateur rotatif puis purifié par flash-chromatographie sur colonne de silice ($CH_2Cl_2$ / MeOH 99 : 1) pour donner :

**Intermédiaire I-7b : 9-chloro-4-(N-BOC-1-aminoéthane)-5,10-dihydropyrido[2,3-g]quinoline-5,10 dione**

- 70 mg (Rendement : 6 %) sous forme de poudre marron.
- $^1H$ RMN ($CDCl_3$) : 1,35 (s, 9H) ; 3,45-3,52 (m, 4H) ; 4,86 (s large, 1H) ; 7,56 (d, 1H, J = 4,0 Hz) ; 7,74 (d, 1H, J = 5,2 Hz) ; 8,90 (d, 1H, J = 5,2 Hz) ; 8,94 (d, 1H, J = 4 Hz) ; • $^{13}C$ RMN ($CDCl_3$) : 28,37 ; 35,32 ; 40,30 ; 79,47 ; 126,84 ; 128,04 ; 130,88 ; 131,17 ; 145,78 ; 150,34 ; 150,98 ; 152,29 ; 154,05 ; 154,36 ; 155,88 ; 179,76 ; 182,32.
- IR ($CHCl_3$) : 1695 $cm^{-1}$.

**Intermédiaire II-7b : 6-chloro-4-(N-BOC-1-aminoéthane)-5,10-dihydropyrido[3,2-g]quinoline-5,10-dione**

- 200 mg (Rendement : 17 %) sous forme d'une poudre marron.
- $^{13}C$ RMN ($CDCl_3$) : 28,24 ; 34,96 ; 40,33 ; 79,47 ; 128,46 ; 130,15 ; 131,06 ; 131,59 ; 145,20 ; 148,76 ; 149,71 ; 151,74 ; 153,88 ; 153,92 ; 155,84 ; 179,76 ; 183,20.
- IR ($CHCl_3$) : 1705 $cm^{-1}$.

**B-6 : Synthèse de la 3-méthoxy-4-méthylpyrido[2,3-g]quinoline-5,10-dione (intermédiaire I-8b) et de la 3-méthoxy-4-méthylpyrido[3,2-g]quinoline-5,10-dione (intermédiaire II-8b)**

Un mélange de 1 g (6,28 mmol) de quinoline-5,8-dione, 1,78 g (12,57 mmol) de la diméthylhydrazone du 2-méthoxy-2-buténal dans 25 ml de $CHCl_3$ sont agités à température ambiante pendant 5 heures. Après évaporation du solvant à l'évaporateur rotatif, le milieu réactionnel est filtré sur silice ($CH_2Cl_2$/MeOH 95 : 5) pour donner 1,55 g de mélange des deux isomères I-8a et II-8a sous forme de poudre violette. Cette poudre et 1 g (11,5 mmol) de $MnO_2$ sont mis en suspension dans 30 ml de $CHCl_3$ et le mélange est agité à température ambiante pendant 1 heure. Après filtration sur célite, le filtrat est concentré à l'évaporateur rotatif puis purifié par flash-chromatographie sur colonne de silice ($CH_2Cl_2$/MeOH 99 : 1) pour donner :

**Intermédiaire I-8b : la 3-méthoxy-4-méthylpyrido[2,3-g]quinoline-5,10-dione**

- 110 mg (Rendement : 7 %) sous forme d'une poudre marron.
- Point de fusion : > 260 °C.
- $^{1}H$ RMN ($CDCl_3$) : 2,79 (s, 3H) ; 4,11 (s, 3H) ; 7,72 (dd, 1H, J = 4,8 et 8,1 Hz) ; 8,66 (s , 1H) ; 8,67 (dd, 1H, J = 8,1 et 1,9 Hz) ; 9,10 (dd, 1H, J = 4,8 et 1,9 Hz).
- $^{13}C$ RMN ($CDCl_3$) : 13,03 ; 56,87 ; 127,88 ; 129,50 ; 129,95 ; 135,50 ; 136,64 ; 139,26; 142,56 ; 149,33 ; 155,11 ; 157,24 ; 180,63 ; 183,56.
- IR ($CHCl_3$) : 1684 $cm^{-1}$.

**Intermédiaire II-8b : la 3-méthoxy-4-méthylpyrido[3,2-g]quinoline-5,10-dione**

- 190 mg (Rendement : 12 %) sous forme d'une poudre marron.
- Point de fusion : > 260 °C.
- $^{1}H$ RMN ($CDCl_3$) : 2,77 (s, 3H) ; 4,12 (s, 3H) ; 7,74 (dd, 1H, J = 4,6 et 8,0 Hz) ; 8,60 (dd, 1H, J = 8,0 et 1,6 Hz) ; 8,68 (s, 1H) ; 9,12 (dd, 1H, J = 4,6 et 1,6 Hz).
- $^{13}C$ RMN ($CDCl_3$) : 12,98 ; 56,93 ; 127,99 ; 129,06 ; 131,27 ; 135,53 ; 136,84 ; 138,81 ; 143,27 ; 148,16 ; 155,20; 157,16, 179,69 ; 184,59.
- IR ($CHCl_3$) : 1670 ; 1692 $cm^{-1}$.

**B-7: Synthèse de la 3,9-diméthoxy-4-méthylpyrido[2,3-g]quinoline-5,10-dione (intermédiaire I-9a) et de la 3,6-diméthoxy-4-méthylpyrido[3,2-g]quinoline-5,10-dione (intermédiaire II-9b)**

A une solution de 4-méthoxyquinoline dione (1,33 g, 7 mmol) dans 30 ml de chloroforme, on ajoute goutte à goutte une solution de 2-méthoxy-2-buténal-diméthylhydrazone (1 g, 7,1 mmol) dans 15 ml de chloroforme. Le milieu réactionnel est maintenu sous agitation à température ambiante, sous azote et à l'abri de la lumière pendant 5 heures. Après évaporation du solvant à l'évaporateur rotatif, le brut obtenu est purifié par flash-chromatographie sur silice ($CHCl_3$ puis $CHCl_3$/MeOH, 98 : 2 puis 95 : 5) pour obtenir une première fraction $F_1$ contenant le produit non aromatique et une deuxième fraction $F_2$ contenant le produit attendu. On ajoute 1g de $MnO_2$ à la fraction F1 et 30 ml de chloroforme. On laisse sous agitation 90 min. Après filtration sur célite et lavage du précipité au $CHCl_3$ puis au MeOH, on concentre à l'évaporateur rotatif pour obtenir une fraction $F_{1'}$. Les fractions $F_{1'}$ et $F_2$ sont rassemblées puis purifiées par flash-chromatographie sur silice ($CHCl_3$ puis $CHCl_3$/MeOH 97 : 3) pour donner les deux composés I-9a et II-9b attendus sous forme d'une poudre marron .

**Intermédiaire II-9b : la 3,6-diméthoxy-4-méthylpyrido[3,2-g]quinoline-5,10-dione**

- Rendement : 11 % (210 mg).
- Point de fusion : > 260°C.
- $^1H$ RMN ($CDCl_3$) : 2,68 (s, 3H) ; 4,09 (s, 3H) ; 4,10 (s, 3H) ; 7,18 (d, 1H, J = 5,5 Hz) ; 8,60 (s, 1H) ; 8,88 (d, 1H, J = 5,5 Hz).
- $^{13}C$ RMN ($CDCl_3$) : 12,85 ; 56,81 ; 56,84 ; 111,14 ; 121,32 ; 130,95 ; 136,43 ; 137,79 ; 141,95; 150,31 ; 155,44 ; 157,33 ; 165,97 ; 180,13 ; 184,24.
- IR ($CHCl_3$) : 1678, 1692 $cm^{-1}$.

**B-8 - Synthèse de la 3-méthoxy-4-méthyl-9-chloropyrido[2,3-g]quinoline-5,10-dione (intermédiaire I-10b) et de la 3-méthoxy-4-méthyl-6-chloropyrido[3,2-g]quinoline-5,10-dione (intermédiaire II-10b)**

A une solution de 4-chloroquinoline dione (1,37 g, 7,1 mmol) dans 30 ml de chloroforme, une solution de 2-méthoxy-2-buténal-diméthylhydrazone (1 g, 7,1 mmol) dans 15 ml de chloroforme est ajoutée goutte à goutte. Le milieu réactionnel est maintenu sous agitation à température ambiante, sous azote et à l'abri de la lumière pendant 5h30 . Après évaporation du solvant à l'évaporateur rotatif, le brut obtenu est purifié par flash-chromatographie sur silice ($CHCl_3$ puis $CHCl_3$/MeOH, 98 : 2) pour obtenir une première fraction $F_1$ contenant le produit non aromatique. On ajoute 1g de $MnO_2$ à cette fraction $F_1$ et 30 ml de chloroforme. On laisse sous agitation à température ambiante 60 min. Après filtration sur célite et lavage du précipité au $CHCl_3$ puis au MeOH, le milieu est concentré à l'évaporateur rotatif. Le brut obtenu est purifié par flash-chromatographie sur silice (97 : 3) pour donner les composés I-10b et II-10b sous forme d'une poudre jaune.

**Intermédiaire II-10b : la 3-méthoxy-4-méthyl-6-chloropyrido[3,2-g]quinoline-5,10-dione**

- Rendement : 5 % (100 mg).
- Point de fusion > : 260 °C.
- $^1H$ RMN ($CDCl_3$) : 2,68 (s, 3H) ; 4,11 (s, 3H) ; 7,71 (d, 1H, J = 5,2 Hz) ; 8,64 (s, 1H) ; 8,90 (d, 1H, J = 5,2 Hz).
- $^{13}C$ RMN ($CDCl_3$) : 12,96 ; 56,97 ; 128,92 ; 130,72 ; 130,98 ; 136,95 ; 138,12 ; 141,93 ; 145,06 ; 150,21 ; 153,85 ; 157,55 ; 179,31 ; 183,67.
- IR ($CHCl_3$) : 1696 ; 1684 $cm^{-1}$.

**B-9 : Synthèse de la 3-méthoxy-4-méthyl-9-diméthylaminopyrido[2,3-g]quinoline-5,10-dione (intermédiaire I-11b) et de la 3-méthoxy-4-méthyl-6-diméthylaminopyrido[3,2-g]quinoline-5,10-dione (intermédiaire II-11b)**

Une solution de I-10b ou de II-10b (90 mg, 0,31 mmol), de chlorure de diméthyl ammonium (127 mg, 1,56 mmol) et de NaOH (63 mg, 1,56 mmol) dans un mélange THF/$H_2O$ (4 ml : 2 ml) est portée à reflux pendant 1 heure. Après évaporation du solvant à l'évaporateur rotatif, le brut obtenu est repris dans un mélange $CH_2Cl_2$/MeOH 95 : 5 (50 ml). La phase organique est récupérée puis sèchée sur $MgSO_4$. Après concentration à l'évaporateur rotatif, le brut obtenu est purifié par flash-chromatographie sur silice ($CH_2Cl_2$/MeOH, 95 : 5) pour donner les composés I-11b ou II 11 b attendus sous forme de poudre jaune.

**Intermédiaire II-11b : 3-méthoxy-4-méthyl-6-diméthylaminopyrido[3,2-g]quinoline-5,10-dione**

- Rendement : 87 % (80 mg)
- Point de fusion : > 260 °C.
- $^1H$ RMN ($CDCl_3$) : 2,64 (s, 3H) ; 3,06 (s, 6H) ; 4,08 (s, 3H) ; 6,95 (d, 1H, J = 5,9 Hz) ; 8,53 (d, 1H, J = 5,9 Hz) ; 8,56 (s, 1H).
- $^{13}C$ RMN ($CDCl_3$) : 12,62 ; 43,40 ; 56,80 ; 112,39 ; 120,50 , 132,23 ; 135,90 ; 136,08 ; 141,86 ; 150,53; 151,70; 155,04; 157,19 ; 180,67; 185,45.
- IR ($CHCl_3$) : 1693 ; 1654 $cm^{-1}$.

**B-10 : Synthèse de la 3,7-diméthoxy-4-méthylpyrido[2,3-g]quinoline-5,10-dione (intermédiaire I-12b) et de la 3,8-diméthoxy-4-méthylpyrido[3,2-g]quinoline-5,10-dione (intermédiaire II-12b)**

**1- Synthèse de la 2-méthoxyquinoline-5,8-dione**

Une suspension de 5,8-dioxocarbostyril (3,1 g, 17,7 mmol), de carbonate d'argent (10,2 g, 37 mmol) et d'iodure de méthyle (31 ml, 498 mmol) dans 1,2 l de $CHCl_3$ est agitée dans le noir et à température ambiante pendant 90 heures. Le précipité est éliminé par filtration et le filtrat est concentré à l'évaporateur rotatif. Le brut obtenu est purifié par filtration sur silice ($CHCl_3$) pour donner la quinone attendue sous forme de solide jaune (2,2 g).

- Rendement : 66 %).
- Point de fusion : 196 °C.
- $^1H$ RMN ($CDCl_3$) : 4,14 (s, 3H) ; 6,95 (d, 1H, J = 10,3 Hz) ; 7,02 (d, 1H, J = 10,3 Hz) ; 7,06 (d, 1H, J = 8,8 Hz) ; 8,25 (d, 1H, J = 8,8 Hz).
- $^{13}C$ RMN ($CDCl_3$) : 54,70 ; 116,68 ; 124,32 ; 136,83 ; 137,54 ; 138,21 ; 146,58 ; 167,14 ; 183,48 ; 184,31.

**2- Synthèse de la 3,7-diméthoxy-4-méthylpyrido[2,3-g]quinoline-5,10-dione (intermédiaire 1-12b) et de la 3,8-diméthoxy-4-méthylpyrido[3,2-g]quinoline-5,10-dione (intermédiaire II-12b)**

A une solution de méthoxyquinoline dione (1,0 g, 5,3 mmoles) dans 60 ml de THF, une solution de 2-méthoxy-2-buténal-diméthylhydrazone (0,75 g, 5,3 mmoles) dans 10 ml deTHF est ajoutée goutte à goutte. Le milieu réactionnel est maintenu sous agitation à température ambiante, sous azote et à l'abri de la lumière pendant 40 heures. Après évaporation du solvant à l'évaporateur rotatif, le brut obtenu est solubilisé dans 80 ml de $CHCl_3$ et du $MnO_2$ 85 % (5,4 g, 53 mmol) est ajouté. Le milieu réactionnel est maintenu sous agitation pendant 2 heures puis filtré sur célite. Après concentration à l'évaporateur rotatif, le brut obtenu est purifié par flash-chromatographie sur silice ($CHCl_3$) pour donner les composés I-12b et II-12b sous forme d'une poudre marron.

**Intermédiaire II-12b : 3,8-diméthoxy-4-méthylpyrido[3,2-g]quinoline-5,10-dione**

- Rendement : 8 % (120 mg)
- Point de fusion : > 260°C.
- $^1H$ RMN ($CDCl_3$) : 2,74 (s, 3H) ; 4,09 (s, 3H) ; 4,20 (s, 3H) ; 7,09 (d, 1H, J = 8,4 Hz) ; 8,41 (d, 1H, J = 8,4 Hz) ; 8,63 (s, 1H).
- $^{13}C$ RMN ($CDCl_3$) :
- IR ($CHCl_3$) : 1667, 1693 $cm^{-1}$.

**B-11 : Synthèse de la 8-éthoxycarbonyl-6-(2'-N-BOC-aminoéthyl)pyrido[2,3-g]quinoline-5,10-dione (intermédiaire I-13b) et de la 7-éthylcarbonyle-6-(2'-N-BOC-aminoéthyl)pyrido[3,2-g]quinoline-5,10-dione (intermédiaire II-13b)**

A une solution de 3-éthylquinolinecarboxylate-5,8-dione (1,05 g, 4,54 mmol) et d'anhydride acétique (4,6 ml) dans 75 ml d'acétonitrile, on ajoute goutte à goutte une solution de N-BOC-5-amino-2-penten-1-al-diméthylhydrazone (1,1g, 4,56 mmol) dans 15 ml d'acétonitrile. Le milieu réactionnel est maintenu sous agitation à température ambiante, sous azote et à l'abri de la lumière pendant 24 heures. Après évaporation du solvant à l'évaporateur rotatif on ajoute 5 g de $MnO_2$ et 150 ml de chloroforme au brut obtenu. On laisse sous agitation à température ambiante 1h30. Après filtration sur célite et lavage du précipité au $CHCl_3$ puis au MeOH, le milieu est concentré à l'évaporateur rotatif. Le brut obtenu est purifié d'abord par filtration sur silice ($CH_2Cl_2$/MeOH 99 : 1 puis 97 : 3) puis par flash-chromatographie sur silice (99 : 1) pour donner les composés I-13b et II-13b sous forme d'une poudre marron.

**Intermédiaire II-13b : la 7-éthoxycarbonyl-6-(2'-N-BOC-aminoéthyl)pyrido[3,2-g]quinoline-5,10-dione**

- Rendement : 3 %(60 mg).
- Point de fusion : 170 °C.
- $^1H$ RMN ($CDCl_3$) : 1,36 (s, 9H) ; 1,47 (t, 3H, J = 7,4 Hz) ; 3,52 (m, 4H) ; 4,51 (q, 2H, J= 7,4 Hz) ; 4,78 (slarge, 1H) ;

7,57 (d, 1H, J = 5,2 Hz) ; 8,99 (d, 1H, J = 5,2 Hz) ; 9,17 (d, 1H, J = 2,2 Hz) ; 9,64 (d, 1H, J = 2,2 Hz).

- $^{13}$C RMN ($CDCl_3$) : 14,33 ; 28,40 ; 35,74 ; 40,22 ; 62,62 ; 79,63 ; 128,65 ; 130,33 ; 130,49 ; 131,83 ; 137,30 ; 149,60 ; 150,23 ; 152,72 ; 154,23 ; 155,72 ; 155.98 ; 163,52 ; 179,69; 183,38.
- IR ($CHCl_3$) : 3457 ; 1726 ; 1705 ; 1677 $cm^{-1}$.

**B-12 : Synthèse de la 7-hydroxy-4-(2'-N-Boc-aminoéthyl)pyrido[2,3-g]quinoline-5,10-dione (intermédiaire I-14b) et de la 8-hydroxy-4-(2'-N-Boc-aminoéthyl)pyrido[3,2-g]quinoline-5,10-dione (intermédiaire II-14b)**

A une solution de 5,8-dioxocarbostyril (0,98 g, 5,59 mmol) et d'anhydride acétique (5,8 ml) dans 100 ml d'acétonitrile, une solution de N-BOC-5-amino-2-penten-1-al-diméthylhydrazone (1,49 g, 6,15 mmol) dans 30 ml d'acétonitrile est ajoutée goutte à goutte. Le milieu réactionnel est maintenu sous agitation à température ambiante, sous azote et à l'abri de la lumière pendant 16 heures. Après évaporation du solvant à l'évaporateur rotatif 7 g (80,5 mmol) de $MnO_2$ et 180 ml de chloroforme sont ajoutés au brut obtenu. Le milieu est laissé sous agitation à température ambiante pendant 1h30. Après filtration sur célite et lavage du précipité au $CHCl_3$ puis au MeOH, le milieu est concentré à l'évaporateur rotatif. Le brut obtenu est purifié par filtration sur silice ($CH_2Cl_2$/MeOH 98 : 2 puis 95 : 5) pour donner le composé I-14b et II-14b sous forme d'une poudre marron.

**Intermédiaire II-14b : la 8-hydroxy-4-(2'-N-Boc-aminoéthyl)pyrido[3,2-g]quinoline-5,10-dione**

- Rendement : 12 % (230 mg).
- Point de fusion : 252 °C.
- $^{1}$H RMN ($CDCl_3$) : 1,56 (s, 9H) ; 3,49 (m, 4H) ; 4,73 (slarge, 1H) ; 6,94 (d, 1H, J = 9,6 Hz) ; 7,54 (d, 1H, J = 4,8 Hz) ; 8,10 (d, 1H, J = 9,6 Hz) ; 8,89 (d, 1H, J = 4,8 Hz) ; 9,66 (slarge, 1H).
- $^{13}$C RMN ($CDCl_3$) : 28,29 ; 35,53 ; 40,24 ; 117,01 ; 127,87 ; 128,62 ; 132,29 ; 136,18 ; 138,04; 148,25 ; 152,26 ; 153,33 ; 155,86 ; 176,36 ; 181,35.
- IR ($CHCl_3$) : 3457 ; 3340 ; 1693 ; 1663 $cm^{-1}$.

**B-13: Synthèse de la 7-hydroxy-4-méthylpyrido[2,3-g]quinoline-5,10-dione (intermédiaire I-15b) et de la 8-hydroxy-4-méthylpyrido[3,2-g]quinoline-5,10-dione (intermédiaire II-15b)**

A une solution de 5,8-dioxocarbostyril (1 g, 5,71 mmol) et d'anhydride acétique (6,2 ml) dans 220 ml d'acétonitrile, une solution de 2-buténal-diméthylhydrazone (0,703 g, 6,28 mmol) dans 20 ml d'acétonitrile est ajoutée goutte à goutte. Le milieu réactionnel est maintenu sous agitation à température ambiante, sous azote et à l'abri de la lumière pendant 16 heures puis chauffé à reflux pendant 6 heures . Après évaporation du solvant à l'évaporateur rotatif, le brut obtenu est purifié par filtration sur silice ($CH_2Cl_2$ puis $CH_2Cl_2$/MeOH, 98 : 2) pour obtenir une première fraction contenant le produit non aromatique et le produit attendu. 3 g de $MnO_2$ et 75 ml de chloroforme sont ajoutés au milieu qui est laissé sous agitation à température ambiante pendant une nuit. Après filtration sur célite et lavage du précipité au $CHCl_3$ puis au MeOH, le milieu est concentré à l'évaporateur rotatif. Le brut obtenu est purifié par flash-chromatographie sur silice (99 : 1) pour donner les composés I-15b et II-15b attendus sous forme d'une poudre beige.

**Intermédiaire II-15b : la 8-hydroxy-4-méthylpyrido[3,2-g]quinoline-5,10-dione.**

- Rendement : 12 %.
- Point de fusion : > 260 °C.
- $^{1}$H RMN (DMSO-$d_6$) : 2,79 (s, 3H) ; 6,82 (d, 1H, J = 9,5 Hz) ; 7,73 (d, 1H, J = 5,2 Hz) ; 8,05 (d, 1H, J = 9,5 Hz) ; 8,85 (d, 1H, J = 5,2 Hz) ; 12,27 (slarge, 1H).
- $^{13}$C RMN (DMSO-$d_6$): 21,92 ; 114,30 ; 122,66 ; 127,30 ; 131,52 ; 135,94 ; 148,60 ; 149,80 ; 152,48 (2C) ; 176,41 ; 182,13 (2C) .
- IR ($CHCl_3$) : 1684 ; 1664 $cm^{-1}$.

**B-14: Synthèse de la 7-méthoxy-4-méthylpyrido[2,3-g]quinoline-5,10-dione (intermédiaire I-16b) et de la 8-méthoxy-4-méthylpyrido[3,2-g]quinoline-5,10-dione (intermédiaire II-16b)**

Un mélange de composé I-15b ou II-15b (70 mg, 0,29 mmol), de iodure de méthyle (1ml, 15,9 mmol) et de

$Ag_2CO_3$ (170 mg, 0,62 mmol) dans 100 ml de $CHCl_3$, est agité à température ambiante et à l'abri de la lumière pendant 14 heures puis chauffé à 56 °C pendant 5 heures. Après concentration à l'évaporateur rotatif, le brut obtenu est purifié par flash-chromatographie sur silice ($CH_2Cl_2$/MeOH 99,5 : 0,5) pour donner les composés I-16b ou II-16b attendus sous forme de poudre beige-marron .

**Intermédiaire II-16b : 8-méthoxy-4-méthylpyrido[3,2-g]quinoline-5,10-dione.**

- Rendement: 41 % (30 mg).
- Point de fusion : 128 °C.
- $^1$ H RMN ($CDCl_3$) : 4,14 (s, 3H) ; 7,07 (d, 1H, J = 8.8 Hz) ; 7,44 (d, 1H, J = 4,8 Hz) ; 8,37 (d, 1H, J = 8,8 Hz) ; 8.85 (d, 1H, J = 4,8 Hz).
- $^{13}$C RMN ($CDCl_3$) : 54,92 ; 117,58 ; 126,24 ; 128,09 ; 131,30 ; 137,73; 147,31 ; 150,00 ; 151,34 ; 153,38 ; 167,39 ; 180,44 ; 183,70.
- IR ($CHCl_3$) : 1765 ; 1698 ; 1667 ; 1603 $cm^{-1}$.

**B-15: Synthèse de la 7,9-dichloro-4-méthylpyrido[2,3-g]quinoline-5,10-dione (intermédiaire I-17b) et de la 6,8-dichloro-4-méthylpyrido[3,2-g]quinoline-5,10-dione (intermédiaire II-17b)**

**1. Synthèse de la 2,4-dichloroquinoline-5,8-dione**

A une solution de 2,4-dichloro-5,8-diméthoxyquinoline (2,85 g, 11,04 mmol) dans un mélange $CH_3CN/H_2O$ (150 ml / 75 ml), du nitrate de cérium ammonium (CAN 21,4 g, 39,03 mmol) est ajouté par portion. Le milieu réactionnel est agité à température ambiante pendant 40 min. L'acétonitrile est ensuite évaporé et 50 ml d'eau et 200 ml d'une solution saturée de $NaHCO_3$ sont ajoutés. La phase aqueuse est extraite au CH2Cl2 (5 fois 200 ml). Après séchage sur $MgSO_4$, le solvant est évaporé à l'évaporateur rotatif pour donner le composé attendu sous forme d'une poudre marron (1,9 g).

- Rendement : 75 %.
- Point de fusion : 161 °C.
- $^1$H RMN ($CDCl_3$) : 7,03 (d, 1H, J = 10,6 Hz) ; 7,11 (d, 1H, J = 10,6 Hz) ; 7,74 (s, 1H).
- $^{13}$C RMN ($CDCl_3$) 124,43 ; 131,10 ; 136,91 ; 139,52 ; 146,69 ; 148,96 ; 156,16 ; 180,53 ; 182,01 .
- IR ($CHCl_3$) : 1687 ; 1676 $cm^{-1}$.

**2. Synthèse de la 7,9 dichloro-4-méthylpyrido[2,3-g]quinoline-5,10-dione (intermédiaire I-17b) et de la 6,8 dichloro-4-méthylpyrido[3,2-g]quinoline-5,10-dione (intermédiaire II-17b)**

A une solution de 2,4-dichloroquinoline-5,8 dione (0,6 g, 2,63 mmol) et d'anhydride acétique (5 ml) dans 100 ml d'acétonitrile, une solution de 2-buténal-diméthylhydrazone (0,325 g, 2,89 mmol) dans 20 ml d'acétonitrile est ajoutée goutte à goutte. Le milieu réactionnel est maintenu sous agitation à température ambiante, sous azote et à l'abri de la lumière pendant 20 heures. Après évaporation du solvant à l'évaporateur rotatif, le brut obtenu est repris dans 140 ml de $CHCl_3$. Sont ajoutés ensuite 3,65 g de $MnO_2$, puis le milieu est laissé sous agitation à température ambiante pendant 56 heures. Après filtration sur célite et lavage du précipité au $CHCl_3$ puis au MeOH, la solution est concentrée à l'évaporateur rotatif. Le brut obtenu est purifié par flash-chromatographie sur silice ($CH_2Cl_2$) pour donner les composés 1-17b et II-17b attendus sous forme d'une poudre marron .

**Intermédiaire II-17b : 6,8 dichloro-4-méthylpyrido[3,2-g]quinoline-5,10-dione.**

- Rendement: 41 % (314 mg).
- Point de fusion : 177 °C.
- $^1$H RMN ($CDCl_3$) : 2,87 (s, 3H) ; 7,56 (d, 1H, J = 4,8 Hz) ; 7,79 (s, 1H) ; 8,93 (d, 1H, J = 4,8 Hz).
- $^{13}$C RMN ($CDCl_3$) : 22,41 ; 125,44 ; 127,84 ; 131,13 ; 131,30 ; 147,44 ; 149,81 ; 150,62 ; 151,90; 154,30 ; 156,58; 179,12; 180,66.
- IR ($CHCl_3$) : 1706 ; 1683 $cm^{-1}$

**B 16 - Synthèse de la 7,9-diméthoxy-4-méthylpyrido[2,3-g]quinoline-5,10-dione (intermédiaire I-18b) et de la 6,8-diméthoxy-4-méthylpyrido[3,2-g]quinoline-5,10-dione (intermédiaire II-18b)**

Un mélange de composé I-17b ou de composé II-17b (80 mg, 0,27 mmol) et de méthylate de sodium (300 mg de Na dans 40 ml de méthanol, 13,04 mmol) dans 40 ml de méthanol est porté à reflux pendant 17 heures. Le milieu réactionnel est concentré à sec puis 50 ml d'eau sont ajoutés. Après neutralisation par HCl 25 %, la solution est extraite au $CH_2Cl_2$ (3 fois 50 ml). Après séchage sur MgSO4 et évaporation du solvant à l'évaporateur rotatif, les composés I-18b ou II-18b attendus quantitativement.

**Intermédiaire II-18b : 6,8-diméthoxy-4-méthylpyrido[3,2-g]quinoline-5,10-dione.**

- Point de fusion : 219 °C.
- $^1$H RMN ($CDCl_3$) : 2,88 (s, 1H) ; 4,03 (s, 3H) ; 4,07 (s, 3H) ; 6,53 (s, 1H), 7,45 (d, 1H, J = 4,8 Hz) ; 8,83 (d, 1 H, J = 4,8 Hz).
- $^{13}$C RMN ($CDCl_3$) : 22,64 ; 54,73; 56,80 ; 97,79 ; 117,61 ; 129,55; 131,46; 148,67 ; 149,41 ; 150,73 ; 152,96 ; 167,95 ; 168,00 ; 180,91 ; 183,41.
- IR ($CHCl_3$) : 1701 ; 1668 $cm^{-1}$.

<u>**EXEMPLE 1**</u>

***7H*-pyrido[4,3,2-*de*][1,10]phénanthroline-7-one (CRL 8293) et*7H*-pyrido[4,3,2-*de*][1,7]phénanthroline-7-one (CRL 8294)**

63 mg (2,81 mmol) de composé I-1b et 1,7 ml (9,84 mmol) de diméthylformamide diéthylacétal dans 4,5 ml de DMF sont portés à 120°C, sous azote, pendant 1 heure. Après évaporation du solvant à la pompe à vide, 3,5 g (65 mmol) de $NH_4Cl$ et 60 ml d'éthanol absolu sont ajoutés. Le milieu réactionnel est porté à reflux pendant 30 min. Après évaporation de l'éthanol à l'évaporateur rotatif, le résidu est additionné de 50 ml d'eau et extrait au $CH_2Cl_2$ (3 fois 50 ml). Après séchage des phases organiques sur $MgSO_4$ et évaporation du solvant à l'évaporateur rotatif, 0,6 g de CRL8294 sont obtenus sous forme d'une poudre verdâtre.

***7H*-pyrido[4,3,2-*de*][1,10]phénanthroline-7-one (CRL 8293)**

- Rendement : 90 %.
- Point de fusion : 240 °C.
- $^1$H RMN ($CDCl_3$) : 7,68 (dd, 1H, J = 4,4 et 8 Hz) ; 7,87 (d, 1H, J = 5,6 Hz) ; 8,02 (d, 1H, J = 5,2 Hz) ; 8,77 (dd, 1H, J = 1,6 et 8 Hz) ; 9,11 (d, 1H, J = 5,2 Hz) ; 9,16 (dd, 1H, J = 1,6 et 4,4 Hz) ; 9,19 (d, 1H, J = 5,6 Hz).
- $^{13}$C RMN ($CDCl_3$) : 120,95 ; 124,40 ; 126,14 ; 129,32 ; 136,78 ; 139,09 ; 147,45 ; 148,58 ; 148,82 ; 148,96 ; 150,66 ; 152,00; 155,73 ; 181,96.

***7H*-pyrido[4,3,2-*de*][1,7]phénanthroline-7-one (CRL 8294)**

En suivant la procédure décrite, ci-dessus, à partir de l'intermédiaire II-1b, 72 mg de composé CRL 8294 sont obtenus sous forme d'une poudre jaune.

- Rendement : 80 %.
- $^1$H RMN ($CDCl_3$) :7,76 (dd, 1H, J = 4,4 et 8 Hz) ; 7,80 (d, 1H, J = 5,2 Hz) ; 7,99 (d, 1H, J = 5,6 Hz) ; 8,93 (d, 1H, J = 5,6 Hz) ; 9,05 (dd, 1H, J = 1,6 et 4,4 Hz) ; 9,17 (dd, 1H, J = 1,6 et 8 Hz) ; 9,19 (d, 1H, J = 5,2 Hz).
- $^{13}$C RMN ($CDCl_3$) : 119,39 ; 120,01 ; 123,85 ; 128,15 ; 132,87 ; 133,80 ; 138,65 ; 147,54 ; 147,74 ; 148,93 ; 149,49 ; 149,99 ; 152,97 ; 180,73.

**EXEMPLE 2**

**8-méthoxy-*7H*-pyrido[4,3,2-*de*][1,10]phénanthroline-7-one (CRL 8363) et11-méthoxy-*7H*-pyrido[4,3,2-*de*][1,7] phénanthroline-7-one (CRL 8364).**

74 mg (2,92 mmol) du composé I-2b et 2 ml (11,8 mmol) de diméthylformamide diéthylacétal dans 5,2 ml de DMF sont portés à 120°C, sous azote, pendant 1 heure. Après évaporation du solvant à la pompe à vide, 4,5 g (83,6 mmol) de $NH_4Cl$ et 67 ml d'éthanol absolu sont ajoutés. Le milieu réactionnel est porté à reflux pendant 30 min. Après évaporation de l'éthanol à l'évaporateur rotatif, le résidu est additionné de 50 ml d'eau et extrait au $CH_2Cl_2$ (3 fois 50 ml). Après séchage des phases organiques sur $MgSO_4$ et évaporation du solvant à l'évaporateur rotatif, le résidu est purifié par flash-chromatographie sur colonne de silice ($CHCl_3$ / MeOH 98 : 2) pour donner 0,28 g de composé CRL 8363 sous forme d'une poudre orange.

**8-méthoxy-*7H*-pyrido[4,3,2-de][1,10]phénanthroline-7-one (CRL 8363).**

- Rendement : 37%.
- $^1$H RMN ($CDCl_3$) : 4,20 (s, 3H) ; 7,13 (d, 1H, J = 5,6 Hz) ; 7,82 (d, 1H, J = 5,2 Hz) ; 7,94 (d, 1H, J = 6 Hz) ; 8,92 (d, 1H, J = 5,6 Hz) ; 9,07 (d, 1H, J = 6 Hz) ; 9,13 (d, 1H, J = 5,2 Hz) ; 9,19 (d, 1H, J = 5,2 Hz).
- $^{13}$C RMN ($CDCl_3$) : 56,77 ; 109,26 ; 119,70 ; 120,47 ; 123,09 ; 138,50 ; 147,85 ; 148,25 ; 148,69 ; 150,66 ; 154,08 ; 155,68 ; 167,54 ; 180,40.

**11-méthoxy-*7H*-pyrido[4,3,2-*de*][1,7]phénanthroline-7-one (CRL 8364)**

En suivant la procédure décrite, ci-dessus, à partir de 1,14 g de l'intermédiaire II-2b, 0,59 g de composé CRL 8364 sont obtenus sous forme d'une poudre jaune.

- Rendement : 50%.
- $^1$H RMN ($CDCl_3$) : 4,15 (s, 3H) ; 7,26 (d, 1H, J = 6 Hz) ; 7,70 (d, 1H, J = 6 Hz) ; 7,96 (d, 1H, J = 5,6 Hz) ; 8,85 (d, 1H, J = 6 Hz) ; 8,97 (d, 1H, J = 6 Hz) ; 9,15 (d, 1H, J = 5,6 Hz).
- $^{13}$C RMN ($CDCl_3$) : 57,05 ; 111,33 ; 118,72 ; 119,61 ; 122,12 ; 124,29 ; 138,56 ; 146,71 ; 147,10; 148,69 ; 149,81 ; 150,96 ; 153,13 ; 165,83 ; 180,82.

**EXEMPLE 3**

**8-(diméthylamino)-*7H*-pyrido[4,3,2-*de*][1,10]phénanthroline-7-one (CRL8800) et11-(diméthylamino)-*7H*-pyrido [4,3,2-*de*][1,7]phénanthroline-7-one (CRL8367)**

80 mg (0,3 mmol) de tricycle I-3b ou de tricycle II-3b et 0,21 ml (1,05 mmol) de diméthylformamide diéthylacétal dans 1,2 ml de DMF sont portés à 120°C, sous azote, pendant 1 heure. Après évaporation du solvant à la pompe à vide, 0,5 g (9,3 mmol) de $NH_4Cl$ et 80 ml d'éthanol absolu sont ajoutés. Le milieu réactionnel est porté à reflux pendant 40 min. Après évaporation de l'éthanol à l'évaporateur rotatif, le résidu est additionné de 5 ml d'eau et extrait au $CH_2Cl_2$ (3 fois 5 ml). Après séchage des phases organiques sur $MgSO_4$ et évaporation du solvant à l'évaporateur rotatif, les 2 composés tétracycliques sont obtenus quantitativement sous forme d'une poudre rouge.

**11-(diméthylamino)-*7H*-pyrido[4,3,2-*de*][1,7]phénanthroline-7-one (CRL8367)**

- $^1$H RMN ($CDCl_3$) : 3,00 (s, 6H) ; 7,09 (d, 1H, J = 5,2 Hz) ; 7,57 (d, 1H, J = 5,6 Hz) ; 7,90 (d, 1H, J = 5,2 Hz) ; 8,54 (d, 1H, J = 5,2 Hz) ; 8,89 (d, 1H, J = 5,2 Hz) ; 9,11 (d, 1H, J = 5,6 Hz).

## EXEMPLE 4

**8-hydroxy-*7H*-pyrido[4,3,2-*de*][1,10]phénanthroline-7-one (CRL8802) et11-hydroxy-*7H*-pyrido[4,3,2-*de*][1,7] phénanthroline-7-one (CRL 8388)**

50 mg (0,126 mmol) de tricyle I-7b ou de tricycle II-7b sont mis en solution dans 0,5 ml de TFA, puis le milieu réactionnel est agité 24 heures. Le TFA est évaporé à l'évaporateur rotatif puis on ajoute une solution saturée de $NaHCO_3$ jusqu'à obtention de pH 9-10. Le milieu est extrait au $CH_2Cl_2$ (3 fois 3 ml). Après séchage sur $MgSO_4$ et évaporation du solvant à l'évaporateur rotatif, on obtient 20 mg de composé tétracyclique sous forme de poudre jaune.

**11-hydroxy-*7H*-pyrido[4,3,2-*de*][1,7]phénanthroline-7-one (CRL 8388).**

- Rendement : 62%.
- Point de fusion : > 260 °C.
- $^1H$ RMN ($CDCl_3$) : 7,20 (d, 1H, J = 5,6 Hz) ; 7,83 (d, 1H, J = 6 Hz) ; 8,00 (d, 1H, J = 6 Hz) ; 8,72 (d, 1H, J = 6 Hz) ; 8,76 (d, 1H, J = 6 Hz) ; 9,24 (d, 1H, J = 5,6 Hz), 14,65 (s, 1 H).
- $^{13}C$ RMN (DMSO, $d_6$) : 116,22; 116,35; 118,61 ; 120,24 ; 124,06 ; 138,09 ; 143,61 ; 148,04 ; 148,99 ; 149,41 ; 152,61 ; 153,01 ; 165,80 ; 179,55.

## EXEMPLE 5

**8-chloro-*7H*-pyrido[4,3,2-*de*][1,10]phénanthroline-7-one (CRL 8396) et11-chloro-*7H*-pyrido[4,3,2-*de*][1,7]phénanthroline-7-one (CRL8801)**

260 mg (0,67 mmol) de tricycle I-7b ou de tricycle II-7b sont mis en solution dans 2,6 ml de TFA, puis le milieu réactionnel est agité 64 heures. Le TFA est évaporé à l'évaporateur rotatif puis on ajoute 200 ml de $CH_2Cl_2$/MeOH 95 : 5 et une solution saturée de $NaHCO_3$ jusqu'à obtention de pH 10. On récupère la phase organique qui est lavée à l'eau. Après séchage sur $MgSO_4$ et évaporation du solvant à l'évaporateur rotatif, 40 mg de composés tétracycliques sont obtenus sous forme d'une poudre marron que l'on lave à l'éther.

**8-chloro-7*H*-pyrido[4,3,2-*de*][1,10]phénanthroline-7-one (CRL 8396)**

- Rendement : 28%.
- Point de fusion : > 260 °C.
- $^1H$ RMN ($CDCl_3$) : 7,68 (d, 1H, J = 5,2 Hz) ; 7,89 (d, 1H, J = 5,5 Hz) ; 8,01 (d, 1H, J = 5,5 Hz) ; 8,96 (d, 1H, J = 5,2 Hz) ; 9,14 (d, 1H, J = 5,5 Hz) ; 9,19 (d, 1H, J = 5,5 Hz).
- $^{13}C$ RMN (DMSO, $d_6$) :119,87 ; 120,88 ; 123,61 ; 126,31 ; 129,01 ; 138,56 ; 146,87 ; 147,37 ; 148,46 ; 148,94 ; 149,76 ; 153,85 ; 153,96 ; 179,87.

## EXEMPLE 6

**4-méthoxy-*7H*-pyrido[4,3,2-*de*][1,10]phénanthroline-7-one (CRL 8400) et4-méthoxy-*7H*-pyrido[4,3,2-*de*][1,7] phénanthroline-7-one (CRL 8401)**

100 mg (0,39 mmol) de tricycle I-8b ou de tricycle II-8b et 0,27 ml (1,37 mmol) de diméthylformamide diéthylacétal dans 0,7 ml de DMF sont portés à 120 °C, sous azote, pendant 1 heure. Après évaporation du solvant à la pompe à vide, 0,6 g (11,7 mmol) de $NH_4Cl$ et 90 ml d'éthanol absolu sont ajoutés. Le milieu réactionnel est porté à reflux pendant 30 min. Après évaporation de l'éthanol à l'évaporateur rotatif, le résidu est additionné de 10 ml d'eau et extrait au $CH_2Cl_2$ (3 fois 10 ml). Après séchage des phases organiques sur $MgSO_4$, évaporation du solvant à l'évaporateur rotatif et purification par filtration sur silice ($CH_2Cl_2$ / MeOH 95: 5) les composés CRL 8400 et CRL 8401 sont obtenus sous forme d'une poudre marron.

**4-méthoxy-*7H*-pyrido[4,3,2-*de*][1,10]phénanthroline-7-one (CRL 8400)**

- Rendement : 83% (85 mg).
- Point de fusion : > 260 °C.
- $^{1}H$ RMN ($CDCl_3$) : 4,27 (s, 3H) ; 7,65 (dd, 1H, J = 4,8 et 8 Hz) ; 8,15 (d, 1H, J = 6 Hz) ; 8,70 (s, 1H) ; 8,78 (dd, 1H, J = 8 et 1,9 Hz) ; 9,10 (d, 1H, J = 6 Hz) ; 9,13 (dd, 1H, J = 1,9 et 4,8 Hz).
- $^{13}C$ RMN (DMSO, $d_6$) :56,97 ; 115,63 ; 120,81 ; 125,52 ; 129,02 ; 129,16 ; 130,22 ; 136,24 ; 139,81 ; 147,37 ; 149,31 ; 151,65 ; 153,07 ; 154,81 ; 180,34.
- IR ($CHCl_3$) : 1674 $cm^{-1}$.

**4-méthoxy-*7H*-pyrido[4,3,2-*de*][1,7]phénanthroline-7-one (CRL 8401)**

- Rendement : 59 %.
- Point de fusion : > 260 °C.
- $^{1}H$ RMN ($CDCl_3$): 4,27 (s, 3H) ; 7,74 (dd, 1H, J = 4,4 et 8,1 Hz) ; 8,08 (d, 1H, J = 5,6 Hz) ; 8,72 (s, 1H) ; 8,93 (d, 1H, J = 5,6 Hz) ; 9,05 (dd, 1H, J = 1,9 et 4,4 Hz) ; 9,19 (dd, 1H, J = 1,9 et 8,1 Hz).
- $^{13}C$ RMN (DMSO, $d_6$): 57,03 ; 115,16 ; 119,70 ; 127,69 ; 129,48 ; 130,15; 132,86; 133,74 ; 140,82 ; 146,80 ; 147,98 ; 148,63 ; 152,81 ; 152,98 ; 179,84.
- IR ($CHCl_3$) : 1679 $cm^{-1}$.

## EXEMPLE 7

**4,8-diméthoxy-7H-pyrido[4,3,2-de][1,10]phénanthroline-7-one (CRL8803) et4,11-diméthoxy-7H-pyrido [4,3,2-de][1,7]phénanthroline-7-one (CRL 8440)**

Une solution du composé I-9b ou du composé II 9b (100 mg, 0,35 mmol) et de N,N-diméthylformamide diéthylacétal (0,24 ml, 1,23 mmol) dans 1ml de DMF est portée à 120 °C pendant 90 min. Le milieu réactionnel est concentré sous vide poussé pour éliminer le DMF et le résidu est dilué dans 100 ml d'EtOH absolu. Après addition de 0,6 g de $NH_4Cl$, le milieu est porté à reflux pendant 30 min. Après concentration à l'évaporateur rotatif, 30 ml d'eau sont ajoutés et le milieu extrait au $CHCl_3$ (3 fois 75 ml). Les phases organiques sont séchées sur $MgSO_4$ et concentrées. Le brut obtenu est purifié par flash-chromatographie sur silice ($CHCl_3$/MeOH 95 : 5) pour donner les composés sous forme de poudre jaune.

**4,11-diméthoxy-7H-pyrido[4,3,2-de][1,7]phénanthroline-7-one (CRL 8440)**

- Rendement : 26 % (27 mg)..
- Point de fusion : > 260 °C.
- $^{1}H$ RMN (DMSO-$d_6$) : 4,08 (s, 3H) ; 4,26 (s, 3H) ; 7,54 (d, 1H, J = 5,9 Hz) ; 7,98 (d, 1H, 5,9 Hz) ; 8,77 (d, 1H, J = 5,9 Hz) ; 8,83 (s, 1H) ; 8,94 (d, 1H, J = 5,9 Hz).
- $^{13}C$ RMN (DMSO-$d_6$) : 57,41 ; 58,07 ; 112,43 ; 113,75 ; 119,84 ; 122,13 ; 129,60 ; 130,54 ; 140,17 ; 146,81 ; 150,17 ; 150,62 ; 153,03; 153,35 ; 166,06 ; 179,30.
- IR ($CHCl_3$) : 1682 ; 1608 ; 1572 $cm^{-1}$.
- MS : m/z 293 (34) ; 292 (42) ; 220 (19) ; 192 (30) ; 165 (22).

## EXEMPLE 8

**4-méthoxy-8-diméthylamino-7H-pyrido[4,3,2-de][1,10]phénanthroline-7-one (CRL8804) et 4-méthoxy-11-diméthylamino-7H-pyrido[4,3,2-de][1,7]phénanthroline-7-one (CRL 8441)**

Une solution du composé I-11b ou du composé II-11b (80 mg, 0,27 mmol) et de N,N-diméthylformamide diéthylacétal (0,18 ml, 0,94 mmol) dans 2 ml de DMF est portée à 120 °C pendant 3 heures. Le milieu réactionnel est concentré sous vide poussé pour éliminer le DMF et le résidu est dilué dans 90 ml d'EtOH absolu. Après addition de

0,4 g de $NH_4Cl$, le milieu est porté à reflux pendant 30 min puis concentré à l'évaporateur rotatif. 30 ml d'eau sont ajoutés puis la solution extraite au $CH_2Cl_2$ (3 fois 50 ml). Les phases organiques sont séchées sur $MgSO_4$ et concentrées. Le brut obtenu est purifié par flash-chromatographie sur silice ($CH_2Cl_2$/MeOH 95 : 5) pour donner les composés tétrecycliques sous forme de poudre rouge-marron.

**4-méthoxy-11-diméthylamino-7H-pyrido[4,3,2-de][1,7]phénanthroline-7-one (CRL 8441)**

- Rendement : 40 % (33 mg).
- Point de fusion : se décompose.
- $^1$H RMN ($CDCl_3$) : 3,02 (s, 6H) ; 4,23 (s, 3H) ; 7,08 (d, 1H, J = 5,9 Hz) ; 7,87 (d, 1H, J = 5,5 Hz) ; 8,54 (d, 1H, J = 5,9 Hz) ; 8,65 (s, 1H) ; 8,90 (d, 1H, J = 5,5 Hz).
- $^{13}$C RMN ($CDCl_3$) : 44,28 ; 56,94 ; 112,14 ; 113,63 ; 119,38 ; 119,73 ; 129,31 ; 129,99; 140,20 ; 145,81 ; 150,31 ; 150,63 ; 151,41 ; 152,99 ; 156,77 ; 180,57.
- IR ($CHCl_3$) : 1682 $cm^{-1}$.
- MS : m/z 306 (52) ; 305 (32) ; 291 (100) ; 290 (66) ; 276 (24) ; 248 (9) ; 220 (13) ; 193 (21).

## EXEMPLE 9

**4,10-diméthoxy-7H-pyrido[4,3,2-de][1,10]phénanthroline-7-one (CRL8805) et4,9-diméthoxy-7H-pyrido [4,3,2-de][1,7]phénanthroline-7-one (CRL 8479)**

Une solution du composé I-12b ou de composé II-12b (100 mg, 0,35 mmol) et de N,N-diméthylformamide diéthylacétal (0,24 ml, 1,23 mmol) dans 1ml de DMF est porté à 120°C pendant 1 heure. Le milieu réactionnel est concentré sous vide poussé pour éliminer le DMF et le résidu est dilué dans 100 ml d'EtOH absolu. Après addition de 0,54 g de $NH_4Cl$, le milieu est porté à reflux pendant 30 min. Après concentration à l'évaporateur rotatif, 20 ml d'eau sont ajoutés et la solution extraite au $CHCl_3$ (3 fois 30 ml). Les phases organiques sont séchées sur $MgSO_4$ et concentrées. Le brut obtenu est purifié par flash-chromatographie sur silice ($CHCl_3$) pour donner les composés tétracycliques sous forme de poudre verte .

**4,9-diméthoxy-7H-pyrido[4,3,2-de][1,7]phénanthroline-7-one (CRL 8479)**

- Rendement : 36 % (37 mg)..
- Point de fusion : > 260 °C.
- $^1$H RMN (DMSO-$d_6$) : 4,21 (s, 3H) ; 4,24 (s, 3H) ; 7,16 (d, 1H, J = 8,8 Hz) ; 7,98 (d, 1H, 5,6 Hz) ; 8,69 (s, 1H) ; 8,85 (d, 1H, J = 5,6 Hz) ; 9,00 (d, 1H, J = 8,8 Hz).
- $^{13}$C RMN (DMSO-$d_6$) : 54,44 ; 56,92 ; 114,04 ; 117,17 ; 118,86 ; 127,74 ; 129,43 ; 129,99 ; 136,29 ; 141,16 ; 146,36 ; 146,72 ; 149,38 ; 152,94 ; 165,80 ; 179,70.
- IR ($CHCl_3$) : 1679 $cm^{-1}$.
- MS : m/z 293 (44) ; 248 (100) ; 220 (12).

## EXEMPLE 10

**9-éthoxycarbonyl-7H-pyrido[4,3,2-de][1,10]phénanthroline-7-one (CRL8805) et10-éthoxycarbonyl-7H-pyrido [4,3,2-de][1,7]phénanthroline-7-one (CRL 8482)**

Une solution du composé I-13b ou du composé II-13b (30 mg, 0,07 mmol) et d'acide trifluoroacétique (0,27 ml, 3,5 mmol) dans 15 ml de $CH_2Cl_2$ est agitée pendant 64 heures. Après concentration à l'évaporateur rotatif, le milieu réactionnel est alcalinisé par 10 ml de solution saturée de $NaHCO_3$ et extrait au $CHCl_3$ (2 fois 30 ml). Les phases organiques sont séchées sur $MgSO_4$ puis concentrées à l'évaporateur rotatif. Le résidu obtenu est purifié par filtration sur silice pour donner les composés tétracycliques sous forme de poudre jaune.

**10-éthoxycarbonyl-7H-pyrido[4,3,2-de][1,7]phénanthroline-7-one (CRL 8482)**

- Rendement : 53 % (11,3 mg).
- Point de fusion : 246 °C.
- $^1H$ RMN ($CDCl_3$) : 1,49 (t, 3H, J = 7,3 Hz) ; 4,53 (q, 2H, J = 7,3 Hz) ; 7,85 (d, 1H, J = 5,9 Hz) ; 8,03 (d, 1H, J = 5,5 Hz) ; 8,98 (d, 1H, J = 5,9 Hz) ; 9,22 (d, 1H, J = 5,5 Hz) ; 9,56 (d, 1H, J = 1,9 Hz) ; 9,73 (d, 1H, J = 1,9 Hz).
- $^{13}C$ RMN ($CDCl_3$) : 14,32 ; 62,29 ; 119,61 ; 120,39 ; 124,04 ; 129,94 ; 132,60 ; 135,46 ; 138,77 ; 147,74 ; 148,78 ; 149,17 ; 149,46 ; 153,23 ; 164,15 ; 180,20 (1C non observé).
- IR ($CHCl_3$) : 1726,1694 $cm^{-1}$.
- MS : m/z .305(92) ; 260 (7) ; 232 (93), 204 (25).

**EXEMPLE 11**

**10-hydroxy-7-H-pyrido[4,3,2-de][1,10]phénanthroline-7-one (CRL8809) et9-hydroxy-7-H-pyrido[4,3,2-de][1,7] phénanthrotine-7-one (CRL 8483)**

Une solution du composé I-14b ou du composé II-14b (tricycle 56) (50 mg, 0,135 mmol) et d'acide trifluoroacétique (0,54 ml, 7 mmol) dans 30 ml de $CH_2Cl_2$ est agitée pendant 48 heures. Après concentration à l'évaporateur rotatif, le milieu réactionnel est alcalinisé par 13 ml de solution saturée de $NaHCO_3$ et extrait au $CH_2Cl_2$ (7 fois 30 ml). Les phases organiques sont séchées sur $MgSO_4$ puis concentrées à l'évaporateur rotatif. Le résidu obtenu est purifié par flash-chromatographie ($CH_2Cl_2$/MeOH 97 : 2) pour donner les composés tétracycliques sous forme de poudre orange.

**9-hydroxy-7-H-pyrido[4,3,2-de][1,7]phénanthroline-7-one (CRL 8483)**

- Rendement : 50 %(16,8 mg)
- Point de fusion : > 260 °C.
- $^1H$ RMN ($CDCl_3$) : 7,06 (d, 1 H, J = 9,5 Hz) ; 7,72 (d, 1H, J = 5,9 Hz) ; 8,02 (d, 1H, J = 5,2 Hz) ; 8,70 (d, 1H, J = 9,5 Hz) ; 8,87 (d, 1H, J = 5,9 Hz) ; 9,19 (d, 1H, J = 5,5 Hz).
- IR ($CHCl_3$) 1690 ; 1667 ; 1602 $cm^{-1}$.
- MS : m/z 249 (100) ; 221 (77,6) ; 193 (99,2).

**EXEMPLE 12**

**10-méthoxy-7-H-pyrido[4,3,2-de][1,10]phénanthroline-7-one (CRL8810) et**9-méthoxy-7-H-pyrido[4,3,2-de][1,7] phénanthroline-7-one (CRL 8484)

Une solution du composé I-16b ou du composé II-16b (200 mg, 0,786 mmol) et de N,N-diméthylformamide diéthylacétal (0,47 ml, 2,73 mmol) dans 3,2 ml de DMF est portée à reflux pendant 2heures. Le milieu réactionnel est concentré sous vide poussé pour éliminer le DMF et le résidu est dilué dans 200 ml d'EtOH absolu. Après addition de 1,4 g de $NH_4Cl$ (26,2 mmol) la solution est portée à reflux pendant 30 min. Après concentration à l'évaporateur rotatif, 50 ml d'eau sont ajoutés puis la solution est extraite au $CH_2Cl_2$ (5 fois 40 ml). Les phases organiques sont séchées sur $MgSO_4$ et concentrées. Le brut obtenu est purifié par flash-chromatographie sur silice ($CH_2Cl_2$/MeOH 99 : 1) pour donner les composés tétracycliques sous forme de poudre marron.

**9-méthoxy-7-H-pyrido[4,3,2-de][1,7]phénanthroline-7-one (CRL 8484)**

- Rendement : 10 %(20 mg).
- Point de fusion : > 260 °C.
- $^1H$ RMN ($CDCl_3$) : 4,14 (s, 3H) ; 7,11 (d, 1H, J = 8,8 Hz) ; 7,63 (d, 1H, J = 5,5 Hz) ; 7,87 (d, 1H, J = 5,5 Hz) ; 8,77 (d, 1H, J = 5,5 Hz) ; 8,91 (d, 1H, J = 8,8 Hz) ; 9,09 (d, 1H, J = 5,5 Hz).
- $^{13}C$ RMN ($CDCl_3$) : 53,41 ; 117,66 ; 118,54 ; 118,93 ; 123,70 ; 127,73 ; 136,29 ; 138,52 ; 145,95 ; 147,45 ; 148,03 ;

148,83 ; 150,19; 165,86 ; 180,55.

- IR ($CHCl_3$) : 1686 $cm^{-1}$.
- MS : m/z 263 (8,2) ; 233 (25,1) ; 204 (35,4).

**EXEMPLE 13**

**8,10-diméthoxy-7-H-pyrido[4,3,2-de][1,10]phénanthroline-7-one (CRL8811) et9,11-diméthoxy-7-H-pyrido [4,3,2-de][1,7]phénanthroline-7-one (CRL 8485)**

On porte à reflux pendant 1h30, une solution du composé I-18b ou du composé II-18b (105 mg, 0,37 mmol) et de N,N-diméthylformamide diéthylacétal (0,22 ml, 1,29 mmol) dans 1,5 ml de DMF. Le milieu réactionnel est concentré sous vide poussé pour éliminer le DMF puis le résidu est dilué dans 95 ml d'EtOH absolu. 0,7 g de $NH_4Cl$ (13,08 mmol) est ajouté et la solution portée à reflux pendant 30 min. Après concentration à l'évaporateur rotatif, 50 ml d'eau sont ajoutés. La solution est extraite au $CH_2Cl_2$ (5 fois 40 ml). Les phases organiques sont séchées sur $MgSO_4$ et concentrées. Le brut obtenu est purifié par flash-chromatographie sur silice ($CH_2Cl_2$/MeOH 99 : 1) pour donner les composés tétracycliques sous forme de poudre jaune-orangé .

**9,11-diméthoxy-7-H-pyrido[4,3,2-de][1,7]phénanthroline-7-one, (CRL 8485)**

- Rendement : 9 %(10 mg).
- Point de fusion : > 260°C.
- $^1H$ RMN ($CDCl_3$) : 4,12 (s, 3H) ; 4,18 (s, 3H) ; 6,65 (s, 1H) ; 7,64 (d, 1H, J = 5,5 Hz) ; 7,92 (d, 1H, J = 5,5 Hz) ; 8,93 (d, 1H, J = 5,5 Hz) ; 9,14 (d, 1H, J = 5,5 Hz).
- $^{13}C$ RMN ($CDCl_3$) : 54,39 ; 57,02 ; 98,26 ; 117,89; 118,64; 118,86; 124,16; 138,50 ; 146,93 ; 147,09 ; 148,29 ; 148,62 ; 151,50 ; 166,32 ; 167,73 ; 180,65.
- IR ($CHCl_3$) : 1688 $cm^{-1}$.
- MS : m/z 293 (15); 292 (28) ; 233 (24) ; 204 (13) ; 165 (10).

**EXEMPLE 14 :**

**8-diméthylamino-10-chloro-7-H-pyrido[4,3,2-de][1,7]phénanthroline-7-one (CRL8812) et 9-chloro-11-diméthylamino-7-H-pyrido[4,3,2-de][1,7]phénanthroline-7-one (CRL 8486)**

Une solution du composé I-17b ou du composé II-17b (110 mg, 0,375 mmol) et de N,N-diméthylformamide diéthylacétal (0,23 ml, 1,31 mmol) dans 1,1 ml de DMF est portée à reflux pendant 1 h30. Le milieu réactionnel est concentré sous vide poussé pour éliminer le DMF puis le résidu est dilué dans 95 ml d'EtOH absolu. Après addition de 0,7 g de $NH_4Cl$ (13,08 mmol), le milieu est porté à reflux pendant 30 min puis concentré à l'évaporateur rotatif. 50 ml d'eau sont ajoutés puis la solution obtenue est extraite au $CH_2Cl_2$ (5 fois 40 ml). Les phases organiques sont séchées sur $MgSO_4$ et concentrées. Le brut obtenu est purifié par flash-chromatographie sur silice ($CH_2Cl_2$/MeOH 99 : 1) pour donner les composés tétracycliques sous forme de poudre rouge-violette .

**9-chloro-11-diméthylamino-7-H-pyrido[4,3,2-de][1,7]phénanthroline-7-one (CRL8486)**

- Rendement : 3 % (3,3 mg).
- Point de fusion : 246 °C.
- $^1H$ RMN ($CDCl_3$) : 3.04 (s, 6H) ; 7.11 (s, 1H) ; 7,61 (d, 1H, J = 5,5 Hz) ; 7,92 (d, 1H, J = 5,5 Hz) ; 8,90 (d, 1H, J = 5,5 Hz) ; 9,14 (d, 1H, J = 5,5 Hz).
- $^{13}C$ RMN ($CDCl_3$) : 44.39; 113.57; 117.60; 119.00 ; 119.37; 123.99; 138.50 ; 146.51 ; 146.77 ; 148.83 ; 150.68 ; 150.89 ; 153.68 ; 158.21 ; 180.05.
- IR ($CHCl_3$) : 1698 $cm^{-1}$.
- MS : m/z 311 (19) ; 309 (11) ; 296 (89) ; 294 (100) ; 269 (4) ; 267 (1) ; 204 (66).

**EXEMPLE 15**

**4-hydroxy-7H-pyrido[4,3,2-de][1,10]phénanthroline-7-one diiodhydrate (CRL8813) et 4-hydroxy-7H-pyrido [4,3,2-de][1,7]phénanthroline-7-one, diiodhydrate (CRL 8487)**

A une suspension de composé CRL 8400 ou de composé CRL 8401 (50 mg, 0,19 mmol) dans l'acide acétique (4 ml), on ajoute l'acide iodhydrique (57 % dans l'eau: 10 ml, 44,6 mmol). Le mélange est chauffé à 100 °C pendant 21 heures. Après refroidissement, puis filtration, le diiodhydrate des composés attendus est isolé sous forme de poudre violette.

**4-hydroxy-7H-pyrido[4,3,2-de][1,7]phénanthroline-7-one, diiodhydrate (CRL 8487)**

- Rendement : 85 %(82 mg).
- Point de fusion : > 260 °C.
- $^1$H RMN (DMSO-$d_6$) : 6,75 (d, 1H, J = 5,8 Hz) ; 7,42 (slarge, 1H) ; 7,63 (dd, 1H, J = 8,4 et 4,4 Hz) ; 8,20 (d, 1H, J = 5,8 Hz) ; 9,07 (m, 2H).
- IR ($CHCl_3$) : 3037 ; 1647 ; 1635 ; 1617 ; 1604 $cm^{-1}$.

**EXEMPLE 16**

**4-chloro-7H-pyrido[4,3,2-de][1,10]phénanthroline-7-one (CRL 8806) et4-chloro-7H-pyrido[4,3,2-de][1,7]phénanthroline-7-one (CRL 8480)**

Une solution de composé CRL 8813 ou de composé CRL 8487 (45 mg, 0,14 mmol) dans le $POCl_3$ (3,5 ml) est portée à reflux pendant 2 heures. Après évaporation à l'évaporateur rotatif, le milieu est alcalinisé à pH 8 par une solution de $NaHCO_3$ 1N (10 ml) puis on extrait par un mélange 5 % MeOH/$CHCl_3$ (2 x 20 ml). Les phases organiques sont séchées sur $MgSO_4$ puis concentrées à l'évaporateur rotatif. Le résidu obtenu est purifié par flash-chromatographie ($CH_2Cl_2$/MeOH, 99 : 1) pour donner les composés attendus sous forme de poudre marron.

**4-chloro-7H-pyrido[4,3,2-de][1,7]phénanthroline-7-one (CRL 8480)**

- Rendement : 4 % (2 mg).
- Point de fusion : > 260 °C.
- $^1$H RMN ($CDCl_3$) : 7,78 (dd, 1H, J = 4,4 et 8,1 Hz) ; 8,08 (d, 1H, 5,9 Hz) ; 9,03 (d, 1H, J = 5,9 Hz) ; 9,07 (dd, 1H, J = 4,4 et 1,8 Hz) ; 9,18 (s, 1H) ; 9,19 (dd, 1H, J = 1,8 et 8,1 Hz).
- $^{13}$C RMN ($CDCl_3$) : 116,63 ; 119,80 ; 128,25 ; 132,64 ; 134,05 ; 137,03 ; 145,92 ; 147,56 ; 147,78 (2C) ; 148,47 ; 149,93 ; 153,31 ; 180,08.
- IR ($CHCl_3$) : 1692 ; 1608 $cm^{-1}$ ·
- MS : m/z 269 (34) ; 267 (100) ; 232 (60) ; 204 (29).

**EXEMPLE 17**

**4-diméthylamino-7H-pyrido[4,3,2-de][1,10]phénanthroline-7-one (CRL 8807) et4-diméthylamino-7H-pyrido [4,3,2-de][1,7]phénanthroline-7-one (CRL8481)**

Une solution de composé CRL 8806 ou de composé CRL 8480 (14 mg, 0,052 mmol), de chlorhydrate de diméthylamine (24 mg, 0,29 mmol) et de soude (13 mg, 0,32 mmol) dans un mélange THF/$H_2O$ (2 ml/1 ml) est portée à reflux pendant 1h30. Après concentration à l'évaporateur rotatif, le milieu est repris par 15 ml d'eau. Après extraction au $CHCl_3$ (3 x 20 ml), les phases organiques sont séchées sur $MgSO_4$ puis concentrées à l'évaporateur rotatif. Le résidu obtenu est purifié par flash-chromatographie ($CHCl_3$/MeOH, 95 : 5) pour donner les composés attendus sous forme de poudre rouge.

**4-diméthylamino-7H-pyrido[4,3,2-de][1,7]phénanthroline-7-one (CRL8481)**

- Rendement : 63 % (9 mg).
- Point de fusion : > 260 °C.
- $^1$H RMN ($CDCl_3$) 3,34 (s, 6H) ; 7,71 (dd, 1H, J = 4,4 et 8,1 Hz) ; 7,96 (d, 1H, 6,0 Hz) ; 8,62 (s, 1H) ; 8,83 (d, 1H, J = 6,0 Hz) ; 9,04 (dd, 1H, J = 1,5 et 4,4 Hz) ; 9,19 (dd, 1H, J = 1,5 et 8,1 Hz).
- $^{13}$C RMN ($CDCl_3$) 44,06 (2C) ; 117,89 ; 120,40 ; 127,22 ; 129,69 ; 132,59 ; 133,68 ; 135,30 ; 138,51 ; 144,67 ; 146,98 ; 148,14 ; 149,16 ; 152,66 ; 179,55.
- IR ($CHCl_3$) : 1666 $cm^{-1}$.
- MS : m/z 276 (100) ; 249 (11); 204 (1).

Les résultats des essais pharmacologiques *in vitro* et *in vivo,* présentés ci-après, mettent en évidence les propriétés cytotoxiques des composés de formule (I) et (la), ainsi que les doses maximales tolérées (DMT).

**1 - Activité cytotoxique sur des lignées cellulaires tumorales en culture (test MTT)**

L'influence des composés de formule (I) et (la) sur les cellules tumorales a été évaluée à l'aide du test colorimétrique MTT (T. Mosman. J. Immunol Methods 1983 ; 65 : 55-63, J. Carmichael *et al*. Cancer Res. 1987 ; 47 : 936-942).

Le principe du test MTT est basé sur la réduction mitochondriale par les cellules vivantes métaboliquement actives du produit MTT (bromure de 3-(4,5-diméthylthiazol-2-yl)-2,5 diphényltétrazolium) de couleur jaune en un produit de couleur bleue, le formazan. La quantité de formazan ainsi obtenue est directement proportionnelle à la quantité de cellules vivantes présentes dans le ou les puits de culture. Cette quantité de formazan est mesurée par spectrophotométrie.

Les lignées cellulaires sont maintenues en culture monocouche à 37° C dans des boîtes de culture à bouchon fermé contenant du milieu de base MEM 25 MM HEPES (Minimum Essential Medium). Ce milieu est bien adapté à la croissance d'une gamme de cellules variées diploïdes ou primaires de mammifères. Ce milieu est ensuite additionnée :

- d'une quantité de 5% de SVF (Sérum de Veau Foetal) décomplémenté à 56° C pendant 1 heure,
- de 0,6 mg/ml de L-glutamine,
- de 200 IU/ml de pénicilline,
- de 200 µg/ml de streptomycine,
- de 0,1 mg/ml de gentamicine.

Les 12 lignées cellulaires cancéreuses humaines qui ont été utilisées ont été obtenues auprès de l'*American Type Culture Collection* (ATCC, Rockville, MD, USA). Ces 12 lignées cellulaires sont :

- U-373MG (code ATCC : HTB-17) et U-87MG (code ATCC : HTB-14) qui sont deux glioblastomes,
- SW1088 (code ATCC : HTB-12) qui est un astrocytome,
- A549 (code ATCC : CCL-185) et A-427 (code ATCC : HTB-53) qui sont deux cancers du poumon non-à-petites-cellules,
- HCT-15 (code ATCC : CCL-225) et LoVo (code ATCC : CCL-229) qui sont deux cancers colorectaux,
- T-47D (code ATCC : HTB-133) et MCF7 (code ATCC : HTB-22) qui sont deux cancers du sein,
- J82 (code ATCC : HTB-1) et T24 (code ATCC : HTB-4) qui sont deux cancers de la vessie,
- PC-3 (code ATCC : CRL-1435) qui est un cancer de la prostate.

Au plan expérimental : 100 µl d'une suspension cellulaire contenant 20 000 à 50 000 (selon le type cellulaire utilisé) cellules/ml de milieu de culture sont ensemencés en plaques multi-puits de 96 puits à fond plat et sont mis à incuber à 37°C, sous atmosphère comprenant 5% de $CO_2$ et 70% d'humidité. Au bout de 24 heures d'incubation, le milieu de culture est remplacé par 100 µl de milieu frais contenant soit les différents composés à tester à des concentrations variant de $10^{-5}$ à $10^{-10}$ M soit le solvant ayant servi à la mise en solution des produits à tester (condition contrôle). Après 72 heures d'incubation dans les conditions précédentes, le milieu de culture est remplacé par 100 µl d'une solution jaunâtre de MTT dissous à raison de 1 mg/ml dans du RPMI 1640. Les microplaques sont remises à incuber pendant 3 heures à 37°C puis centrifugées pendant 10 minutes à 400 g. La solution jaunâtre de MTT est éliminée et les cristaux de formazan bleu formés au niveau cellulaire sont dissous dans 100 µl de DMSO. Les microplaques sont ensuite mises sous agitation pendant 5 minutes. L'intensité de la coloration bleue résultant donc de la

transformation du produit MTT jaune en formazan bleu par les cellules encore vivantes au terme de l'expérience est quantifiée par spectrophotométrie à l'aide d'un appareil de type *DYNATECH IMMUNOASSAY SYSTEM* aux longueurs d'onde de 570 nm et 630 nm correspondant respectivement aux longueurs d'ondes d'absorbance maximale du formazan et au bruit de fond. Un logiciel intégré au spectrophotomètre calcule les valeurs moyennes de densité optique ainsi que les valeurs de déviation standard (Dév. Std.) et d'erreur standard sur la moyenne (ESM).

L'activité inhibitrice de la croissance cellulaire des composés de formule (I) et (la) sur les différentes lignées cellulaires tumorales a été mesurée en comparaison avec celle du produit naturel. A titre d'exemple, les valeurs des concentrations encadrant les concentrations inhibitrices 50 % (CI 50) obtenues pour chaque composé sont présentées, dans le tableau I, ci-après :

L'ensemble des composés étudiés présente une activité inhibitrice significative de la prolifération cellulaire des 12 lignées tumorales humaines : U-87MG, U-373MG, SW 1088, T24, J82,HCT-15, LoVo, MCF7, T-47D, A549, A-427 et PC-3 avec une CI 50 pouvant être comprise entre $10^{-5}$ et $10^{-9}$M, selon les composés et les lignées tumorales testés.

**2 - Détermination de la dose maximale tolérée (DMT)**

L'évaluation de la dose maximale tolérée a été réalisée chez des souris B6D2F1/Jico âgées de 4 à 6 semaines. Les composés ont été administrés par voie intrapéritonéale à des doses croissantes s'échelonnant de 2,5 à 160 mg/kg. La valeur de la DMT (exprimée en mg/kg) est déterminée à partir de l'observation du taux de survie des animaux sur une période de 14 jours après une administration unique du produit considéré. L'évolution pondérale des animaux est également suivie pendant cette période. Lorsque que la valeur de la DMT est supérieure à 160 mg/kg, la valeur de la DMT est assimilée à 160 mg/kg par défaut.

Les résultats de l'estimation de la dose maximale tolérée (DMT) sont rassemblés dans le tableau II suivant :

TABLEAU II

| Doses Maximales Tolérées | |
|---|---|
| Composés CRL | DMT (mg/kg) |
| CRL8388 (Exemple 4) | 10 |
| CRL8293 (Exemple 1) | 10 |
| CRL8294 (Exemple 1) | 10 |
| CRL8363 (Exemple 2) | 10 |
| CRL8364 (Exemple 2) | 5 |
| CRL8367 (Exemple 3) | 10 |
| CRL8396 (Exemple 5) | 20 |
| CRL8400 (Exemple 6) | > 160 |
| CRL8401 (Exemple 6) | > 160 |
| CRL8440 (Exemple 7) | 20 |
| CRL8441 (Exemple 8) | > 160 |

Les produits de cette famille présentent soit une certaine toxicité directe ou peuvent en être dépourvu et être alors utilisés *in vivo* à des concentrations tissulaires élevées, donc à des posologies fortes.

**3 - Activité antitumorale in vivo**

Les essais ont été réalisés sur les modèles de :

- carcinome mammaire murin MXT hormono-insensible (MXT-HI),
- adénocarcinome mammaire murin MXT hormono-sensible (MXT-HS),
- lymphome L 1210.

Le modèle d'adénocarcinome mammaire murin MXT de Watson C. *et al*. (Cancer Res. 1977 ; 37 : 3344 - 48), greffé sur des souris B6D2F1/JIco âgées de 4 à 6 semaines, est dérivé des canaux galactophores de glande mammaire.

Initialement hormono-sensible (modèle MXT-HS), la tumeur différenciée évolue vers une tumeur hormono-insensible indifférencée (modèle MXT-HI). Les agents dont l'activité antitumorale a été démontrée sur le plan clinique prolongent la survie des animaux porteurs de tumeurs MXT-HI et de tumeurs MXT-HS. C'est le cas par exemple du cyclophosphamide, de l'étoposide ou encore de l'adriamycine.

Le modèle de lymphome L 1210 est un modèle de cellules leucémiques L 1210 d'origine murine greffées en sous cutané chez la souris. Elles donnent naissance, dans 100% des cas, à une tumeur solide sous cutanée (L1210 s.c.) à croissance rapide.

Lorsque la valeur de DMT d'un produit a été déterminée, son activité antitumorale *in vivo* a été caractérisée aux doses de DMT/2, DMT/4 et DMT/8 sur les modèles de l'adénocarcinome mammaire d'origine murine MXT-HS , du carcinome mammaire murin MXT-HI et sur le modèle du lymphome L 1210 sous-cutané.

Dans tous les exemples présentés ci-après, quelque soit le modèle, la condition contrôle est représentée par un lot de 9 ou 15 souris auxquelles est administré pendant 3 semaines consécutives et à raison de 3 administrations (lundi, mercredi et vendredi) par semaine un volume de 0,2 ml de sérum physiologique contenant le solvant utilisé pour dissoudre les différents composés de formule (I) et (Ia) utilisés.

Au cours de ces essais, ont été déterminés soit la croissance tumorale soit le taux de survie des souris:

i)- <u>La croissance tumorale</u> a été estimée en mesurant deux fois par semaine (lundi et vendredi) la surface des tumeurs MXT-HS, MXT-HI ou L 1210 greffées. Cette surface est calculée, en effectuant le produit de la valeur des deux plus grands axes perpendiculaires de la tumeur. La valeur de ces axes est mesurée à l'aide d'un pied à coulisse.

ii)- <u>le taux de survie des souris</u> est calculé sous forme d'un rapport T/C ou :

$$T = \text{(Nombre de jours de survie de la souris médiane du lot de souris traitées)} + \frac{\text{(Souris médiane traitée)} - \text{(Nombre de souris mortes dans les jours qui ont précédé celui de la souris médiane traitée)}}{\text{(Nombre de souris mortes le même jour que la souris médiane traitée)}}$$

$$C = \text{(Nombre de jours de survie de la souris médiane du lot de souris traitées)} + \frac{\text{(Souris médiane traitée)} - \text{(Nombre de souris mortes dans les jours qui ont précédé celui de la souris médiane traitée)}}{\text{(Nombre de souris mortes le même jour que la souris médiane contrôle)}}$$

Ce rapport représente le temps de survie moyen de la souris médiane du lot des souris traitées par rapport au temps de survie moyen de la souris médiane du lot des souris contrôles. Ainsi, une molécule induit une augmentation significative ($P < 0.05$) de la survie des animaux lorsque l'indice T/C excède 130%. Par contre elle présente un effet toxique lorsque cette valeur de T/C est inférieure à 70%.

### <u>3.1. - Carcinome mammaire murin (MXT-HI)</u>

A titre d'exemple, nous présenterons ci-dessous l'influence des deux produits CRL8293 et CRL8294 sur la croissance des tumeurs MXT-HI. Chaque lot de souris greffées avec les tumeurs MXT-HI et relatif à une condition expérimentale donnée comprend 15 animaux.

<u>Traitement 1</u>

Le produit CRL8293 est administré par voie intra-péritonéale . La première injection du produit est réalisée au septième jour post-greffe (J7) à raison de 3 injections par semaine (lundi, mercredi et vendredi) pendant 3 semaines

consécutives et à la dose de 5 mg/kg.

Traitement 2

Le produit CRL8294 est administré par voie intra-péritonéale. La première injection du produit est réalisée au septième jour post-greffe (J7) à raison de 3 injections par semaine (lundi, mercredi et vendredi) pendant 3 semaines consécutives et à la dose de 5 mg/kg.

Dans le tableau III suivant, sont indiquées, en pourcentage, les diminutions (-) ou les augmentations (+) de la surface des tumeurs MXT-HI induites avec les traitements 1 et 2 par rapport à la condition contrôle au 21$^{ème}$ jour après la greffe tumorale, soit après 6 administrations du produit CRL8293 ou du produit CRL8294. Au 21$^{ème}$ jour post-greffe 100% des animaux contrôles sont encore en vie.

TABLEAU III

| Traitements | Surface tumorale (exprimé en %) |
|---|---|
| 1 (CRL8293) | - 33 |
| 2 (CRL8294) | - 36 |

Ces résultats montrent que ces deux produits CRL8293 et CRL8294 induisent une diminution significative de la croissance des tumeurs MXT-HI. Ces résultats montrent que ces produits de formule I et la présentent *in vivo* et sur ce modèle une activité antitumorale intéressante.

**3.2.- Adénocarcinome mammaire murin (MXT-HS)**

A titre d'exemple, nous présenterons ci-dessous l'influence des deux produits CRL8293 et CRL8294 sur la croissance des tumeurs MXT-HS. Chaque lot de souris greffées avec les tumeurs MXT-HS et relatif à une condition expérimentale donnée comprend 9 animaux.

Traitement 10

Le produit CRL8293 est administré par voie intra-péritonéale. La première injection du produit est réalisée au septième jour post-greffe (J7) à raison de 3 injections par semaine (lundi, mercredi et vendredi) pendant 3 semaines consécutives et à la dose de 5 mg/kg.

Traitement 20

Le produit CRL8294 est administré seul par voie intra-péritonéale. La première injection du produit est réalisée au septième jour post-greffe (J7) à raison de 3 injections par semaine (lundi, mercredi et vendredi) pendant 3 semaines consécutives et à la dose de 5 mg/kg.

Dans le tableau IV suivant sont indiquées, en pourcentage, les diminutions (-) ou les augmentations (+) de la surface des tumeurs MXT-HS induites avec les traitements 10 et 20 par rapport à la condition contrôle au 31$^{ème}$ jour après la greffe tumorale, soit après les 9 administrations, prévues dans le protocole expérimental, des 2 produits CRL8293 et CRL8294. Au 31$^{ème}$ jour post-greffe 100% des animaux contrôles sont encore en vie.

TABLEAU IV

| Traitements | Surface tumorale (exprimé en %) |
|---|---|
| 10 (CRL8293) | - 45 |
| 20 (CRL8294) | - 64 |

Ces résultats montrent que ces deux produits CRL8293 et CRL8294 induisent une diminution très hautement significative de la croissance des tumeurs MXT-HS. Ces résultats montrent, comme sur le modèle MXT-HI, que les produits de formule I et la présentent également sur le modèle MXT-HS une activité antitumorale très intéressante.

**3.3.- Lymphome L1210**

A titre d'exemple, nous présenterons ci-dessous l'influence du CRL8294 sur le temps de survie des souris

(tableau V). Chaque lot de souris greffées avec le lymphome L1210 et relatif à une condition expérimentale donnée comprend 9 animaux.

Traitement 100

Le produit CRL8294 est administré seul par voie intra-péritonéale. La première injection du produit est réalisée au septième jour post-greffe (J7) à raison de 3 injections par semaine (lundi, mercredi et vendredi) pendant 3 semaines consécutives et à la dose de 1,25 mg/kg.

Tableau V

| Traitement | T/C (exprimé en %) |
|---|---|
| 100 (CRL8294) | 136 |

Sur le modèle du lymphome L 1210 sous-cutané, le composé CRL8294 de formule (I) présente une activité antitumorale. Cette dernière se caractérise par un allongement significatif du temps de survie moyen de la souris médiane du lot de souris ainsi traitées par rapport au temps de survie moyen de la souris médiane du lot des souris contrôles.

**4 - Ratios tolérance/activité cytotoxique**

Dans le tableau VI suivant, sont présentés les résultats des Cl 50 (en nM) moyennes (calculées à partir des activités cytotoxiques individuelles obtenues sur chacune des 12 lignées tumorales étudiées) et les ratios DMT/CI50 calculés en effectuant le rapport des DMT et des CI50. Ce dernier rapport est exprimé en nombre sans dimension.

TABLEAU VI

| Composés CRL | CI50 (nM) | DMT/CI50 | DMT/CI50* |
|---|---|---|---|
| CRL8388 (Exemple 4) | 6200 | 0,0016 | 1 |
| CRL8293 (Exemple 1) | 1250 | 0,008 | 5 |
| CRL8294 (Exemple 1) | 1450 | 0,007 | 4,4 |
| CRL8363 (Exemple 2) | 500 | 0,02 | 12,5 |
| CRL8364 (Exemple 2) | 270 | 0,019 | 12 |
| CRL8367 (Exemple 3) | 1650 | 0,006 | 3,8 |
| CRL8396 (Exemple 5) | 600 | 0,033 | 20,6 |
| CRL8400 (Exemple 6) | 380 | 0,42 | 262 |
| CRL8401 (Exemple 6) | 53 | 3 | 1870 |
| CRL8440 (Exemple 7) | 10 | 0,42 | 1240 |
| CRL8441 (Exemple 8) | 5000 | 3 | 19.8 |

* : le ratio DMT/CI50 des différents composés a été estimé en prenant comme référence un ratio égal à 1 pour CRL8388.

Les composés de formule (I) et (Ia) présentent une activité antitumorale significative à la fois *in vitro* et *in vivo* dans les conditions expérimentales décrites ci-dessus. *In vitro*, ils inhibent la croissance des cellules tumorales, comme en témoignent les résultats des tests colorimétriques MTT. *In vivo,* ils inhibent de manière significative et considérable la croissance des tumeurs MXT-HI et MXT-HS et augmentent de manière significative le temps de survie moyen de la souris médiane du lot des souris ainsi traitées et greffées avec le lymphome L 1210 par rapport au temps de survie moyen de la souris médiane du lot des souris contrôles.

Grâce à leurs propriétés cytotoxiques, les composés de formules (I) et (Ia) tels que décrits ou sous forme de sels ou solvates pharmaceutiques acceptables, peuvent être utilisés comme principes actifs de médicaments pour traiter les tumeurs cancéreuses et leurs métastases.

Les composés de formules (I) et (Ia) sont généralement administrés en unités de dosage établies soit par $m^2$ de surface corporelle, soit par kg de poids. Les dites unités de dosage sont de préférence formulées dans des compositions pharmaceutiques dans lesquelles le principe actif est mélangé avec un (ou plusieurs) excipient(s) pharmaceutique(s).

Les composés de formule (I) et (Ia) peuvent être utilisés selon la pathologie cancéreuse du sujet à traiter à des doses comprises entre 0,05 et 350 mg/$m^2$ de surface corporelle, de préférence à des doses de 0,5 à 50 mg/$m^2$/jour pour un traitement curatif dans sa phase aiguë en fonction du nombre de cycles de traitement de chaque cure. Pour un traitement d'entretien, on utilisera avantageusement les composés de formules (I) et (Ia) à des doses de 0,05 à 25 mg/$m^2$/jour, de préférence à des doses de 0,1 à 1,5 mg/$m^2$/jour selon le nombre de cycles de traitement de la cure. Ils pourront être associés aux médicaments anti-tumoraux utilisés dans les protocoles validés de polychimiothérapie intensive.

Dans les compositions pharmaceutiques de la présente invention pour l'administration par voie orale, intraveineuse, les principes actifs peuvent être administrés sous formes unitaires d'administration, en mélange avec des supports pharmaceutiques classiques adaptés à la thérapeutique humaine. Les formes unitaires d'administration appropriées comprennent les formes par voie orale telles que les comprimés, éventuellement sécables, ou les gélules, les implants et les formes d'administration intraveineuse.

Pour une administration parentérale (perfusion intraveineuse à débit constant), on utilise des suspensions aqueuses stériles, des solutions salines isotoniques stériles ou des solutions stériles et injectables qui contiennent des agents de dispersion et/ou des agents solubilisants pharmacologiquement compatibles, par exemple le propylèneglycol, le polyéthylèneglycol, ou une β cyclodextrine..

Ainsi, pour préparer une solution aqueuse injectable par voie intraveineuse et destinée à une perfusion réalisée sur 1 à 24 h, on peut utiliser un cosolvant : un alcool tel que l'éthanol, un glycol tel que le polyéthylèneglycol ou le propylèneglycol et un tensioactif hydrophile tel que le Tween 80.

Lorsque l'on prépare une composition solide sous forme de comprimés, on peut ajouter au principe actif, micronisé ou non, un agent mouillant tel que le laurylsulfate de sodium et on mélange le tout avec un véhicule pharmaceutique tel que la silice, la gélatine, l'amidon, le lactose, le stéarate de magnésium, le talc, la gomme arabique ou analogues. On peut enrober les comprimés de saccharose, de divers polymères ou d'autres matières appropriées ou encore les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant tel qu'un glycol ou un ester de glycérol et en incorporant le mélange obtenu dans des gélules molles ou dures.

Le principe actif peut être formulé également sous forme de microcapsules ou microsphères, éventuellement avec un ou plusieurs supports ou additifs.

Le principe actif peut être également présenté sous forme de complexe avec une cyclodextrine, par exemple α-, β- ou γ-cyclodextrine, 2-hydroxypropyl-β-cyclodextrine ou méthyl-β-cyclodextrine.

Les composés de formules (I) et (Ia) seront utilisés dans le traitement de la plupart des tumeurs solides du fait de leurs activités cytotoxiques puissantes, en particulier pour traiter les tumeurs cérébrales, les cancers du poumon, les tumeurs de l'ovaire et du sein, les cancers de l'endomètre, les cancers colo-rectaux, les cancers de la prostate et les tumeurs testiculaires.

## Revendications

1. **1.** Composés de formules :

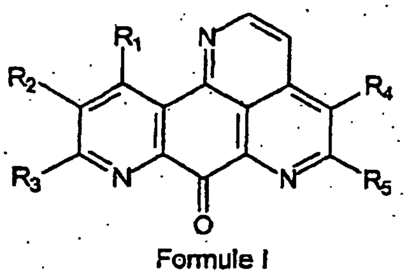

Formule I

et

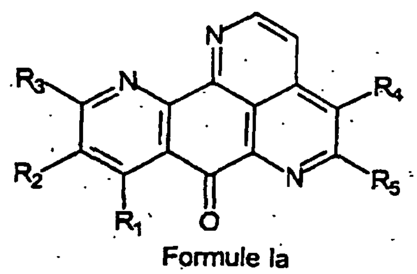

Formule Ia

dans lesquelles :

$R_1$, $R_2$, $R_3$, $R_4$ et $R_5$ sont choisis parmi l'hydrogène, les halogènes, les groupes alkyle en $C_1$-$C_6$, hydroxy, -CHO, $-OR_8$, -COOH, -CN, $-CO_2R_8$, $-CONHR_8$, $-CONR_8R_9$, $-NH_2$, $-NHR_8$, $-N(R_8)_2$ $-NH-CH_2-CH_2-N(CH_3)_2$, $-NH-CH_2-CH_2-Cl$, $-NHCOR_8$, morpholino, nitro, $SO_3H$,

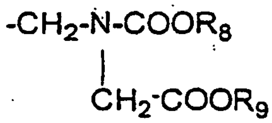 , 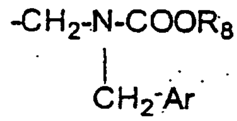

$R_8$ et $R_9$ étant choisis parmi les groupes alkyle en $C_1$-$C_6$, et les groupes phénylalkyle ($C_1$-$C_4$) et Ar étant un groupe aryle en $C_6$-$C_{14}$,
et les sels d'addition de ces composés avec des acides pharmaceutiquement acceptables.

**2.** Composés selon la revendication 1 qui sont des composés de formules I ou la dans lesquelles :

$R_1$, $R_2$, $R_3$, $R_4$ et $R_5$ sont choisis parmi l'hydrogène, les halogènes, les groupes alkyle en $C_1$-$C_6$, hydroxy, -CHO, $-OR_8$, -COOH, -CN, $-CO_2R_8$, $-CONHR_8$, $-CONR_8R_9$, $-NH_2$, $-NHR_8$, $-N(R_8)_2$, $-NH-CH_2-CH_2-N(CH_3)_2$, $-NHCOR_8$, morpholino, nitro , $SO_3H$,

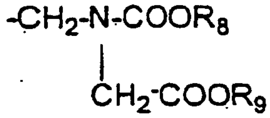 , 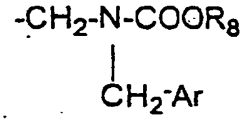

$R_8$ et $R_9$ étant choisis parmi les groupes alkyle en $C_1$-$C_6$, et Ar étant un groupe aryle en $C_6$-$C_{14}$.

**3.** Composés selon la revendication. 1 qui sont des composés de formules I ou la dans lesquelles :

$R_1$, $R_2$, $R_3$, $R_4$ et $R_5$ sont choisis parmi l'hydrogène, les halogènes, les groupes alkyle en $C_1$-$C_6$, hydroxy, $-OR_8$, $NO_2$, $-NH_2$, $-NHR_8$, - $NH(R_8)_2$, $-NH-CH_2-CH_2-N(CH_3)_2$, $-NH-CH_2-CH_2-Cl$, $-NHCOR_8$, $R_8$ étant choisi parmi les groupes alkyle en $C_1$-$C_6$,

et les sels d'addition de ces composés avec des acides pharmaceutiquement acceptables.

**4.** Composés selon la revendication 3 qui sont des composés de formules I ou la dans lesquelles au moins l'un des groupes $R_1$, $R_2$, $R_3$ $R_4$ ou $R_5$ est un groupe $OR_8$.

**5.** Composés selon la revendication 3 qui sont des composés de formules I ou la dans lesquelles :

$R_1$ est choisi parmi l'hydrogène, les halogènes, les groupes hydroxy, méthoxy, nitro, - $NH_2$, $-NHCH_3$, $-NH-CH_2-CH_2-N(CH_3)_2$, $-NH-CH_2-CH_2-Cl$, $-NHCOCH_3$,
$R_2$ est l'hydrogène,
$R_3$ et $R_5$ sont choisis parmi l'hydrogène ou les groupes hydroxy, méthoxy,

et les sels d'addition de ces composés avec des acides pharmaceutiquement acceptables.

**6.** Composés selon la revendication 3 qui sont les composés de formule (I) :

la 11-méthoxy-*7H*-pyrido[4,3,2-*de*][1,7]phénanthroline-7-one,
la 11-chloro-*7H*-pyrido[4,3,2-*de*][1,7]phénanthroline-7-one,
la 4-méthoxy-*7H*-pyrido[4,3,2-*de*][1,7]phénanthroline-7-one,
la 4,11-diméthoxy-7H-pyrido[4,3,2-de][1,7]phénanthroline-7-one,
la 4,9-diméthoxy-7H-pyrido[4,3,2-de][1,7]phénanthroline-7-one,
la 9-méthoxy-7-H-pyrido[4,3,2-de][1,7]phénanthroline-7-one,
la 9,11-diméthoxy-7-H-pyrido[4,3,2-de][1,7]phénanthroline-7-one,
la 3-acétoxyméthyl-7*H*-pyrido[4,3,2-*de*][1,7]phénanthroline-7-one,
la 3-acétoxyméthyl-9-méthoxy-*7H*-pyrido[4,3,2-*de*][1,7]phénanthroline-7-one,
la 2-(2-chloroéthyl)-7*H*-pyrido[4,3,2-*de*][1,7]phénanthroline-7-one,
et les sels d'addition de ces composés avec des acides pharmaceutiquement acceptables.

**7.** Composés selon la revendication 3 qui sont des composés de formule (la):

la 8-méthoxy-7*H*-pyrido[4,3,2-*de*][1,10]phénanthroline-7-one
la 8-chloro-7*H*-pyrido[4,3,2-*de*][1,10]phénanthroline-7-one,
la 4-méthoxy-7*H*-pyrido[4,3,2-*de*][1,10]phénanthroline-7-one,
la 4,8-diméthoxy-7H-pyrido[4,3,2-de][1,10]phénanthroline-7-one,
la 4,10-diméthoxy-7H-pyrido[4,3,2-de][1,10]phénanthroline-7-one,
la 10-méthoxy-7-H-pyrido[4,3,2-de][1,10]phénanthroline-7-one,
la 8,10-diméthoxy-7-H-pyrido[4,3,2-de][1,10]phénanthroline-7-one,
la 3-acétoxyméthyl-7*H*-pyrido[4,3,2-*de*][1,10]phénanthroline-7-one,
la 3-acétoxyméthyl-9-méthoxy-7*H*-pyrido[4,3,2-*de*][1,10]phénanthroline-7-one,
la 2-(2-chloroéthyl)-7*H*-pyrido[4,3,2-*de*][1,10]phénanthroline-7-one,

et les sels d'addition de ces composés avec des acides pharmaceutiquement acceptables.

**8.** Composition pharmaceutique comprenant une quantité efficace d'un composé choisi parmi les composés selon l'une quelconque des revendications 1 à 7, pour traiter, grâce à leurs propriétés cytotoxiques, les tumeurs cancéreuses et leurs métastases.

**9.** Utilisation des composés tels que définis dans l'une quelconque des revendications 1 à 7 pour la fabrication d'un médicament anticancéreux.

**10.** Utilisation selon la revendication 9 pour la fabrication d'un médicament pour le traitement des tumeurs solides.

**11.** Utilisation selon la revendication 10 pour la fabrication d'un médicament pour traiter les tumeurs cérébrales, les cancers du poumon, les tumeurs de l'ovaire et des seins, les cancers de l'endomètre, les cancers colorectaux, les cancers de la prostate et les tumeurs testiculaires.

**12.** Procédé de préparation de composés selon la revendication 1 qui consiste à :

a) faire réagir selon une réaction d'hétéro Diels-Aldar une quinoléine dione de formule :

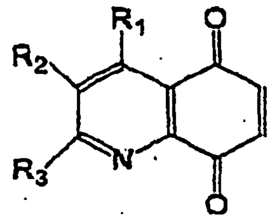

IV

et un azadiène de formule

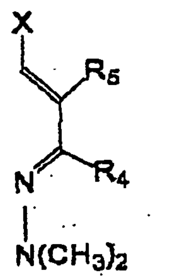

V

où X = $CH_3$
pour obtenir un mélange de composés

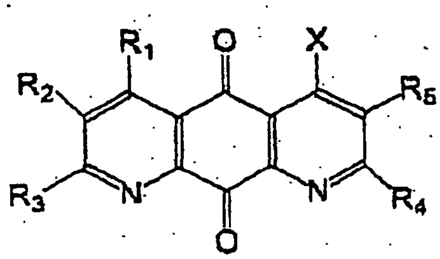

+

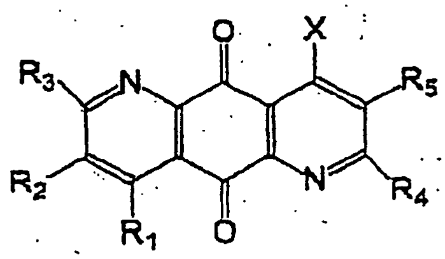

Formule II

Formule IIa

b) éventuellement séparer les composés de formules II et IIa

$c_1$) faire ensuite réagir un composé de formules II et ou IIa avec le diméthylformamide diméthylacetal pour obtenir une ènamine de formule

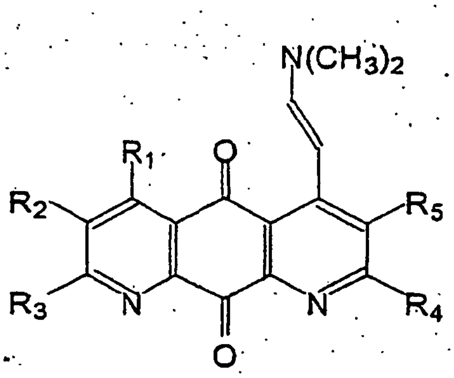

et/ou

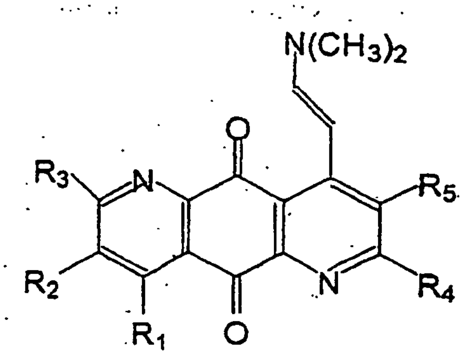

Formule III

Formule IIIa

puis fonctionnaliser les ènamines pour introduire les substituants $R_6$ et/ou $R_7$ et cycliser pour obtenir les composés de formules I et/ou Ia

ou

$c_2$) fonctionnaliser et cycliser en même temps pour obtenir les composés de formules I et/ou Ia,

d) éventuellement séparer les composés de formules I et Ia.

**13.** Procédé de préparation de composés selon la revendication 1 de formules I ou Ia qui consiste

a) à faire réagir selon une réaction d'hétéro Diels-Alder une quinoléine dione de formule :

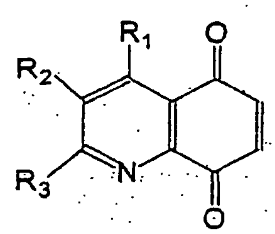

IV

et un azadiène de formule

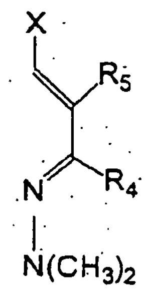

V

où X = $CH_2$-$CH_2$-NHBoc pour obtenir un mélange de composés

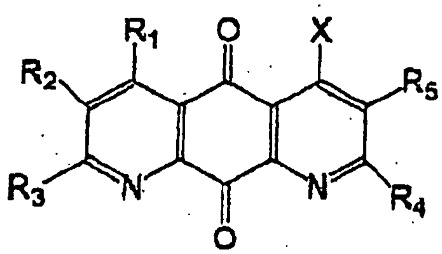

+

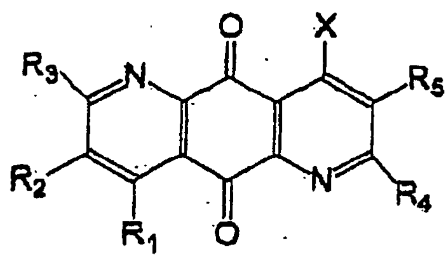

Formule II

Formule IIa

b) éventuellement séparer les composés de formules II et IIa,
c) cycliser un composé de formules II et/ou IIa pour obtenir un composé de formules I et/ou Ia,
d) éventuellement séparer les composés de formules I ou Ia.

**14.** Enamine de formule :

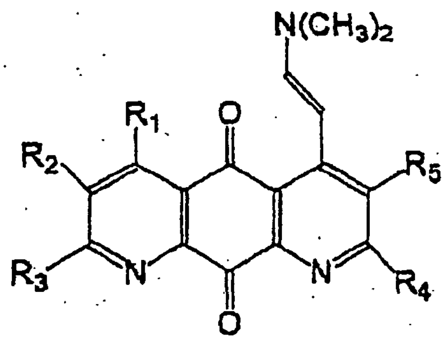

et/ou

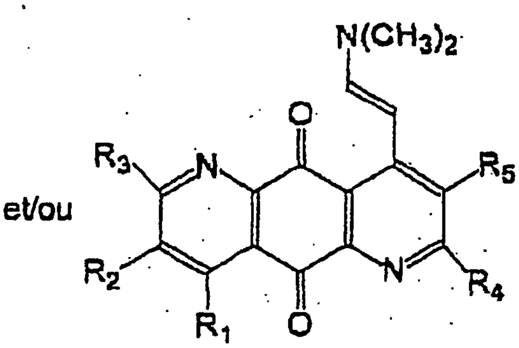

Formule III

Formule IIIa

dans lesquelles :

$R_1$, $R_2$, $R_3$, $R_4$ et $R_5$ sont choisis parmi l'hydrogène, les halogènes, les groupes alkyle en $C_1$-$C_6$, hydroxy, -CHO, -$OR_6$, -COOH, -CN, -$CO_2R_8$, -$CONHR_8$, - $CONR_8R_9$,

-$NH_2$, -$NHR_8$, -$N(R_8)_2$ -NH-$CH_2$-$CH_2$-$N(CH_3)_2$, -NH-$CH_2$-$CH_2$-Cl, -$NHCOR_8$, morpholino, nitro, $SO_3H$,

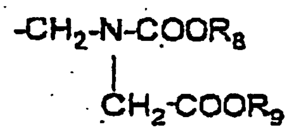 , 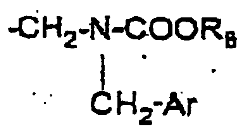

$R_8$ et $R_9$ étant choisis parmi les groupes alkyle en $C_1$-$C_6$, et les groupes phénylalkyle ($C_1$-$C_4$) et Ar étant un groupe aryle en $C_6$-$C_{14}$.

## Patentansprüche

**1.** Verbindungen der Formel

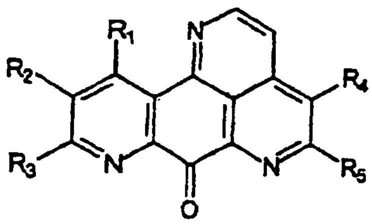 und 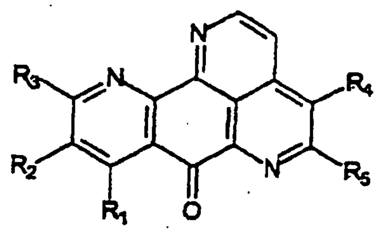

Formel I    Formel Ia

worin
$R_1$, $R_2$, $R_3$, $R_4$ und $R_5$ aus Wasserstoff, Halogen, einer $C_1$-$C_6$-Alkylgruppe, Hydroxy, -CHO, -$OR_8$, -COOH, -CN, -$CO_2R_8$, -$CONHR_8$, -$CONR_8R_9$, -$NH_2$, -$NHR_8$, -$N(R_8)_2$, -NH-$CH_2$-$CH_2$-$N(CH_3)_2$, -NH-$CH_2$-$CH_2$-Cl, -$NHCOR_8$, Morpholino, Nitro, $SO_3H$,

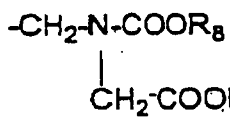 und 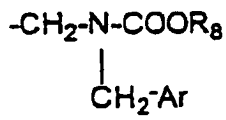

ausgewählt sind,
$R_8$ und $R_9$ aus einer $C_1$-$C_6$-Alkylgruppe und einer Phenyl-$C_1$-$C_4$-alkylgruppe ausgewählt sind und Ar eine $C_6$-$C_{14}$-Arylgruppe ist,
und die Säureadditionssalze dieser Verbindungen mit pharmazeutisch annehmbaren Säuren.

**2.** Verbindungen gemäß Anspruch 1, die Verbindungen der Formel I oder Ia sind, worin
$R_1$, $R_2$, $R_3$, $R_4$ und $R_5$ aus Wasserstoff, Halogen, einer $C_1$-$C_6$-Alkylgruppe, Hydroxy, -CHO, -$OR_8$, -COOH, -CN, -$CO_2R_8$, -$CONHR_8$, -$CONR_8R_9$, -$NH_2$, -$NHR_8$, -$N(R_8)_2$, -NH-$CH_2$-$CH_2$-$N(CH_3)_2$, -$NHCOR_8$, Morpholino, Nitro, $SO_3H$,

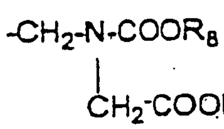 und 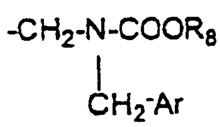

ausgewählt sind,
$R_8$ und $R_9$ aus einer $C_1$-$C_6$-Alkylgruppe ausgewählt sind und Ar eine $C_6$-$C_{14}$-Arylgruppe ist.

**3.** Verbindungen gemäß Anspruch 1, die Verbindungen der Formel I oder Ia sind, worin .
$R_1$, $R_2$, $R_3$, $R_4$ und $R_5$ aus Wasserstoff, Halogen, einer $C_1$-$C_6$-Alkylgruppe, Hydroxy, -$OR_8$, $NO_2$, -$NH_2$, -$NHR_8$, -N

$(R_8)_2$, $-NH-CH_2-CH_2-N(CH_3)_2$, $-NH-CH_2-CH_2-Cl$ und $-NHCOR_8$ ausgewählt sind und $R_8$ aus einer $C_1$-$C_6$-Alkylgruppe ausgewählt ist und die Säureadditionssalze dieser Verbindungen mit pharmazeutisch annehmbaren Säuren.

**4.** Verbindungen gemäß Anspruch 3, die Verbindungen der Formel I oder Ia sind, worin wenigstens eine der Gruppen $R_1$, $R_2$, $R_3$, $R_4$ oder $R_5$ eine Gruppe $OR_8$ ist.

**5.** Verbindungen gemäß Anspruch 3, die Verbindungen der Formel I oder Ia sind, worin $R_1$ aus Wasserstoff, Halogen und den Gruppen Hydroxy, Methoxy, Nitro, $-NH_2$, $-NHCH_3$, $-NH-CH_2-CH_2-N(CH_3)_2$, $-NH-CH_2-CH_2-Cl$ und $-NHCOCH_3$ ausgewählt ist, $R_2$ Wasserstoff ist und $R_3$ und $R_5$ aus Wasserstoff oder den Gruppen Hydroxy und Methoxy ausgewählt sind und die Säureadditionssalze dieser Verbindungen mit pharmazeutisch annehmbaren Säuren.

**6.** Verbindungen gemäß Anspruch 3, die Verbindungen der Formel (I) sind:

11-Methoxy-7H-pyrido[4,3,2-de][1,7]phenanthrolin-7-on,
11-Chlor-7H-pyrido[4,3,2-de][1,7]phenanthrolin-7-on,
4-Methoxy-7H-pyrido[4,3,2-de][1,7]phenanthrolin-7-on,
4,11-Dimethoxy-7H-pyrido[4,3,2-de][1,7]phenanthrolin-7-on,
4,9-Dimethoxy-7H-pyrido[4,3,2-de][1,7]phenanthrolin-7-on,
9-Methoxy-7H-pyrido[4,3,2-de][1,7]phenanthrolin-7-on,
9,11-Dimethoxy-7H-pyrido[4,3,2-de][1,7]phenanthrolin-7-on,
3-Acetoxymethyl-7H-pyrido[4,3,2-de][1,7]phenanthrolin-7-on,
3-Acetoxymethyl-9-methoxy-7H-pyrido[4,3,2-de][1,7]phenanthrolin-7-on,
2-(2-Chlorethyl)-7H-pyrido[4,3,2-de][1,7]phenanthrolin-7-on

und die Säureadditionssalze dieser Verbindungen mit pharmazeutisch annehmbaren Säuren.

**7.** Verbindungen gemäß Anspruch 3, die Verbindungen der Formel (Ia) sind:

8-Methoxy-7H-pyrido[4,3,2-de][1,10]phenanthrolin-7-on,
8-Chlor-7H-pyrido[4,3,2-de][1,10]phenanthrolin-7-on,
4-Methoxy-7H-pyrido[4,3,2-de][1,10]phenanthrolin-7-on,
4,8-Dimethoxy-7H-pyrido[4,3,2-de][1,10]phenanthrolin-7-on,
4,10-Dimethoxy-7H-pyrido[4,3,2-de][1,10]phenanthrolin-7-on,
10-Methoxy-7H-pyrido[4,3,2-de][1,10]phenanthrolin-7-on,
8,10-Dimethoxy-7H-pyrido[4,3,2-de][1,10]phenanthrolin-7-on,
3-Acetoxymethyl-7H-pyrido[4,3,2-de][1,10]phenanthrolin-7-on,
3-Acetoxymethyl-9-methoxy-7H-pyrido[4,3,2-de][1,10]phenanthrolin-7-on,
2-(2-Chlorethyl)-7H-pyrido[4,3,2-de][1,10]phenanthrolin-7-on

und die Säureadditionssalze dieser Verbindungen mit pharmazeutisch annehmbaren Säuren.

**8.** Pharmazeutische Zusammensetzung, die eine wirksame Menge einer aus den Verbindungen gemäß einem der Ansprüche 1 bis 7 ausgewählten Verbindung umfaßt, dank ihrer zytotoxischen Eigenschaften zum Behandeln von Krebsgeschwülsten und ihren Metastasen.

**9.** Verwendung in einem der der Ansprüche 1 bis 7 definierter Verbindungen zur Herstellung eines Arzneimittels gegen Krebs.

**10.** Verwendung gemäß Anspruch 9 zur Herstellung eines Arzneimittels zur Behandlung solider Tumoren.

**11.** Verwendung gemäß Anspruch 10 zur Herstellung eines Arzneimittels zum Behandeln von Hirntumoren, Lungenkrebs, Ovarial- und Brustkrebs, Endometriumkarzinomen, kolorektalen Karzinomen, Prostatakarzinomen und Hodentumoren.

**12.** Verfahren zur Herstellung von Verbindungen gemäß Anspruch 1, das aus

(a) dem Reagierenlassen gemäß einer Hetero-Diels-Alder-Reaktion eines Chinolindions der Formel

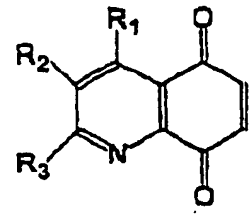

IV

und eines Azadiens der Formel

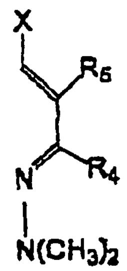

V

worin X = $CH_3$,
unter Erhalten eines Gemisches der Verbindungen

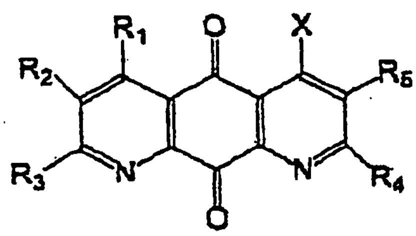

+

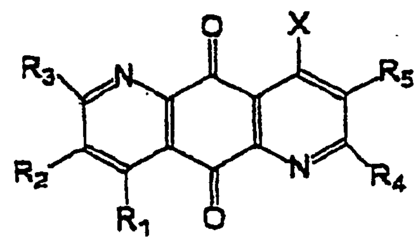

Formel II

Formel IIa

b) gegebenenfalls Trennen der Verbindungen der Formel II und IIa,
$c_1$) anschließend Reagierenlassen einer Verbindung der Formel II oder IIa mit Dimethylformamiddimethylacetal unter Erhalten eines Enamins der Formel

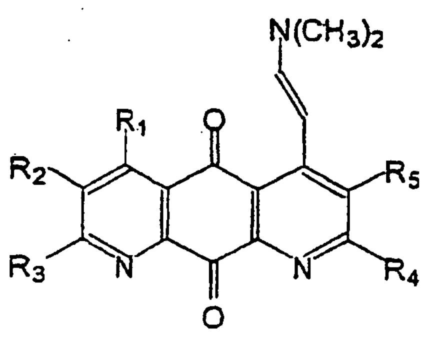

und/oder

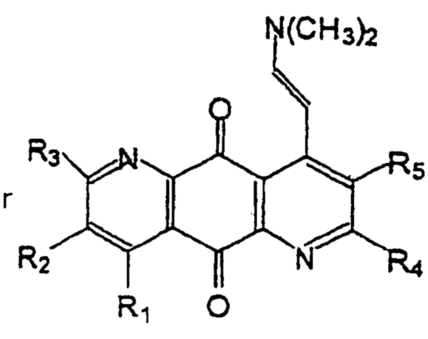

Formel III

Formel IIIa

dann dem Funktionalisieren der Enamine unter Einführen der Substituenten $R_6$ und/oder $R_7$ und Cyclisieren unter Erhalten der Verbindungen der Formel I und/oder Ia
oder

$c_2$) dem gleichzeitigen Funktionalisieren und Cyclisieren unter Erhalten der Verbindungen der Formel I und/oder Ia und
d) gegebenenfalls dem Trennen der Verbindungen der Formel I und Ia besteht.

**13.** Verfahren zur Herstellung von Verbindungen gemäß Anspruch 1 der Formel I oder Ia, das aus

(a) dem Reagierenlassen gemäß einer Hetero-Diels-Alder-Reaktion eines Chinolindions der Formel

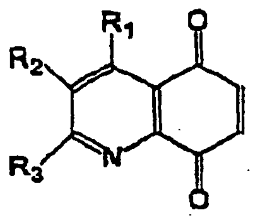

IV

und eines Azadiens der Formel

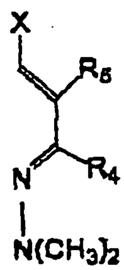

V

worin X = $CH_2$-$CH_2$-NHBoc,
unter Erhalten eines Gemisches der Verbindungen

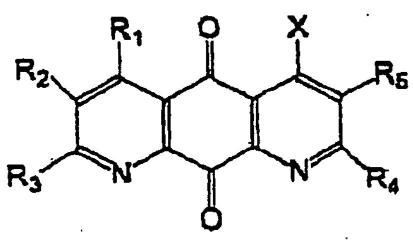

+

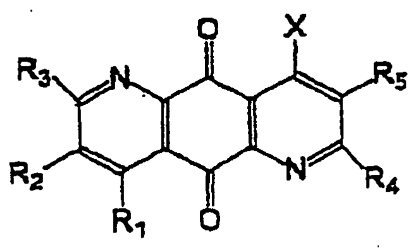

Formel II

Formel IIa

b) gegebenenfalls Trennen der Verbindungen der Formel II und IIa,
c) dem Cyclisieren einer Verbindung der Formel II und/oder IIa unter Erhalten einer Verbindung der Formel I und/oder Ia und
d) gegebenenfalls dem Trennen der Verbindungen der Formel I und Ia besteht.

**14.** Enamin der Formel

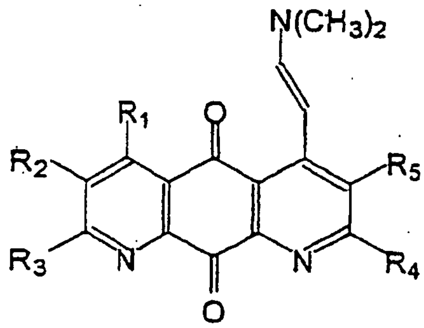

und/oder

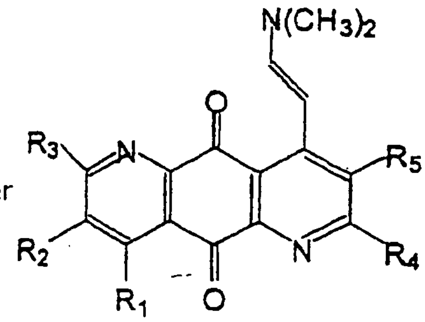

Formel III Formel IIIa

worin
$R_1$, $R_2$, $R_3$, $R_4$ und $R_5$ aus Wasserstoff, Halogen, einer $C_1$-$C_6$-Alkylgruppe, Hydroxy, -CHO, -$OR_8$, -COOH, -CN, -$CO_2R_8$, -$CONHR_8$, -$CONR_8R_9$, -$NH_2$, -$NHR_8$, -$N(R_8)_2$, -NH-$CH_2$-$CH_2$-$N(CH_3)_2$, -NH-$CH_2$-$CH_2$-Cl, -$NHCOR_8$, Morpholino, Nitro, $SO_3H$,

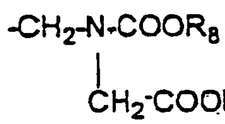

und

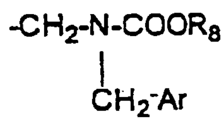

ausgewählt sind,
$R_8$ und $R_9$ aus einer $C_1$-$C_6$-Alkylgruppe und einer Phenyl-$C_1$-$C_4$-alkylgruppe ausgewählt sind und Ar eine $C_6$-$C_{14}$-Arylgruppe ist.

**Claims**

**1.** Compounds of formulas:

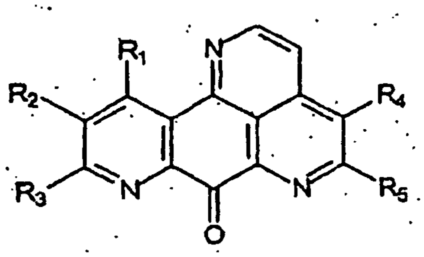

and

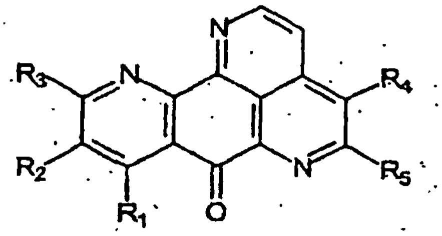

formula I formula Ia

in which:

$R_1$, $R_2$, $R_3$, $R_4$ and $R_5$ are chosen from among hydrogen, halogen, $C_1$-$C_6$ alkyl, hydroxy -CHO, -$OR_8$, -COOH, -CN, -$CO_2R_8$, -$CONHR_8$, -$CONR_8R_9$, -$NH_2$, -$NHR_8$, -$N(R_6)_2$, -NH-$CH_2$-$CH_2$-$N(CH_3)_2$, -NH-$CH_2$-$CH_2$-Cl, -$NHCOR_8$, morpholino, nitro, $HSO_3$ groups,

$-CH_2-N-COOR_8$ | $-CH_2-N-COOR_8$
$\quad\quad\;\; |$ | $\quad\quad\;\; |$
$\quad\quad CH_2-COOR_9$ | $\quad\quad CH_2-Ar$

$R_8$ and $R_9$ being chosen from among $C_1$-$C_6$ alkyl groups, and the ($C_1$-$C_4$) phenyl alkyl groups and Ar being a $C_6$-$C_{14}$ aryl group and the addition salts of these compounds with pharmaceutically acceptable acids.

**2.** Compounds according to claim 1 constituted by compounds of formulas I or in which:

$R_1$, $R_2$, $R_3$, $R_4$ and $R_5$ are chosen from among hydrogen, halogens, $C_1$-$C_6$ alkyl, hydroxy, -CHO, -$OR_8$, -COOH, -CN, -$CO_2R_8$, -$CONHR_8$, -$CONR_8R_9$, -$NH_2$, -$NHR_8$, -$N(R_8)_2$, -NH-$CH_2$-$CH_2$-$N(CH_3)_2$, -$NHCOR_8$, morpholino, nitro, $HSO_3$ groups

$-CH_2-N-COOR_8$ | $-CH_2-N-COOR_8$
$\quad\quad\;\; |$ | $\quad\quad\;\; |$
$\quad\quad CH_2-COOR_9$ | $\quad\quad CH_2-Ar$

$R_8$ and $R_9$ being chosen from among $C_1$-$C_6$ alkyl groups, and Ar being a $C_6$-$C_{14}$ aryl group.

**3.** Compounds according to claim 1, which are compound of formulas I or in which:

$R_1$, $R_2$, $R_3$, $R_4$ and $R_5$ are chosen from among hydrogen, halogens, $C_1$-$C_6$ alkyl, hydroxy, -$OR_8$, $NO_2$, -$NH_2$, -$NHR_8$, -$NH(R_8)_2$, -NH-$CH_2$-$CH_2$-$N(CH_3)_2$, -NH-$CH_2$-$CH_2$-Cl, -$NHCOR_8$, $R_8$ being chosen from among $C_1$-$C_6$ alkyl groups and the addition alts of these compounds with pharmaceutically acceptable acids.

**4.** Compounds according to claim 3, which are compounds of formulas I or in which at least one of the groups $R_1$, $R_2$, $R_3$, $R_4$ or $R_5$ is an $OR_8$ group.

**5.** Compounds according to claim 3, which are compounds of formulas I or in which:

$R_1$ is chosen from among hydrogen, halogens, hydroxyl, methoxy, nitro, -$NH_2$, -$NHCH_3$, -NH-$CH_2$-$CH_2$-N ($CH_3$)$_2$, -NH-$CH_2$-$CH_2$-Cl, -$NHCOCH_3$ groups, $R_2$ is hydrogen, $R_3$ and $R_5$ are chosen from among hydrogen and hydroxy and methoxy groups and the addition salts of these compounds with pharmaceutically acceptable acids.

**6.** Compounds according to claim 3, which are compounds of formula (I):

11-methoxy-7H-pyrido[4,3,2-de][1,7]phenanthroline-7-one,
11-chloro-7H-pyrido[4,3,2-de][1,7]phenanthroline-7-one,
4-methoxy-7H-pyrido[4,3,2-de][1,7]phenanthroline-7-one,
4,11-dimethoxy-7H-pyrido[4,3,2-de][1,7]phenanthroline-7-one,
4,9-dimethoxy-7H-pyrido[4,3,2-de][1,7]phenanthroline-7-one,
9-methoxy-7-H-pyrido[4,3,2-de][1,7]phenanthroline-7-one,
9,11-dimethoxy-7-H-pyrido[4,3,2-de][1,7]phenanthroline-7-one,
3-acetoxymethyl-7H-pyrido[4,3,2-de][1,7]phenanthroline-7-one,
3-acetoxymethyl-9-methoxy-7H-pyrido[4,3,2-de][1,7]phenanthroline-7-one,
2-(2-chloroethyl)-7H-pyrido[4,3,2-de][1,7]phenanthroline-7-one,

and the addition salts of these compounds with pharmaceutically acceptable acids.

**7.** Compounds according to claim 3, which are compounds of formula (Ia):

8-methoxy-7H-pyrido[4,3,2-de][1,10]phenanthroline-7-one,
8-chloro-7H-pyrido[4,3,2-de][1,10]phenanthroline-7-one,

4-methoxy-7H-pyrido[4,3,2-de][1,10]phenanthroline-7-one,
4,8-dimethoxy-7H-pyrido[4,3,2][1,10]phenanthroline-7-one,
4,10-dimethoxy-7H-pyrido[4,3,2-de][1,10]phenanthroline-7-one,
10-methoxy-7-H-pyrido[4,3,2-de][1,10]phenanthroline-7-one,
8,10-dimethoxy-7-H-pyrido[4,3,2-de][1,10]phenanthroline-7-one,
3-acetoxymethyl-7H-pyrido[4,3,2-de][1,10]phenanthroline-7-one,
3-acetoxymethyl-9-methoxy-7H-pyrido[4,3,2-de][1,10]phenanthroline-7-one,
2-(2-chloroethyl)-7H-pyrido[4,3,2-de][1,10]phenanthroline-7-one,

and the addition salts of these compounds with pharmaceutically acceptable acids.

**8.** Pharmaceutical composition comprising an efficaceous quantity of a compound chosen from among the compounds according to any one of the claims 1 to 7, for treatment, as a result of the cytotoxic properties thereof, of cancerous tumour and their metastases.

**9.** Use of the compounds as defined in any one of the claims 1 to 7 for the production of an anticancerous medicament.

**10.** Use according to claim 9 for the production of a medicament for the treatment of solid tumours.

**11.** Use according to claim 10 for the production of a medicament for treating brain tumours, lung, ovarian, breast, endometrium, colorectum, prostate and testicular cancers.

**12.** Process for the preparation of compounds according to claim 1 consisting of:

a) reacting in a hetero Diel-Alder reaction a quinoline dione of formula:

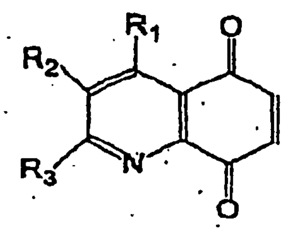

IV

and an azadiene of formula

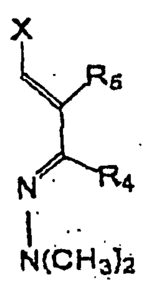

V

where X = $CH_3$
to obtain a mixture of compounds

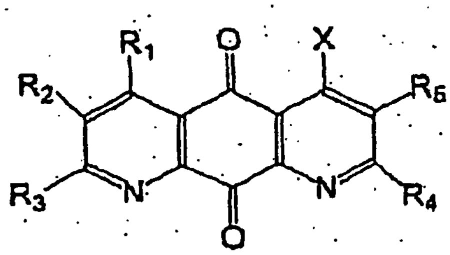
+
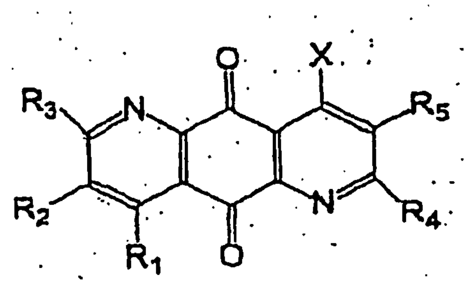

formula II formula IIa

b) optionally separating the compounds of formulas II and IIa

$c_1$) then reacting a compound of formulas II and/or IIa with dimethyl formamide dimethyl acetal to obtain an enamine of formula

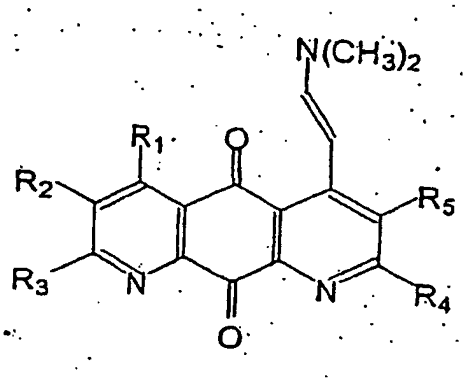
and/or
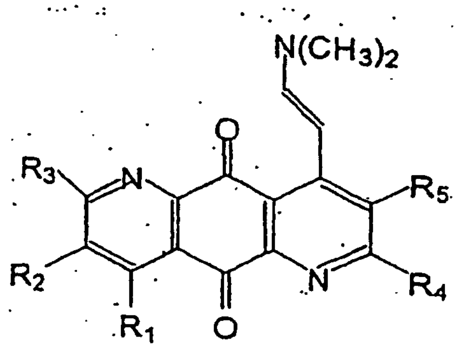

formula III formula IIIa

in order to functionalize the enamines for introducing the substituents $R_6$ and/or $R_7$ and cyclizing to obtain the compounds of formulas I and/or Ia, or

$c_2$) functionalizing and cyclizing at the same time to obtain the compounds of formulas I and/or Ia,

d) optionally separating the compounds of formulas I and Ia.

**13.** Process for the preparation of compounds according to claim 1 of formulas I or Ia and consisting of:

a) reacting in a hetero Diels/Alder reaction a quinoline dione of formula:

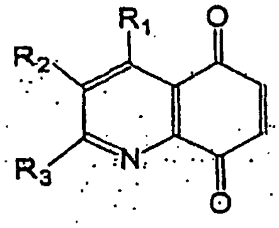

IV

and an azadiene of formula:

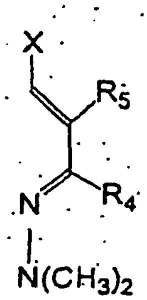

V

in which X = $CH_2$-$CH_2$-NHBoc to obtain a mixture of compounds

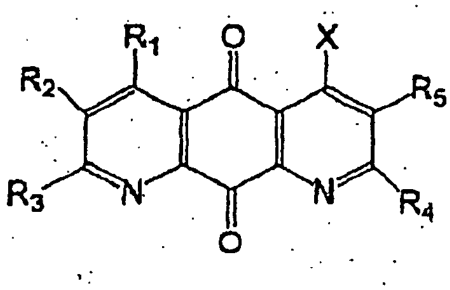

+

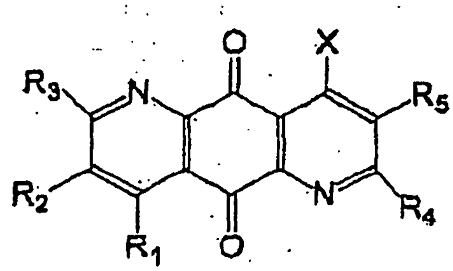

formula II

formula IIa

b) optionally separating the compounds of formulas II and IIa,
c) .cyclizing a compound of formulas II and/or IIa to obtain a compound of formulas I and/or Ia,
d) optionally separating the compounds of formulas I or Ia.

**14.** Enamine of formula:

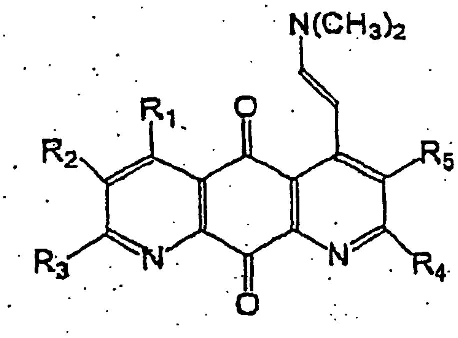 and/or 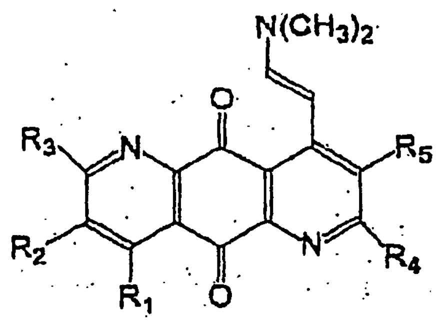

formula III formula IIIa

in which:

$R_1$, $R_2$, $R_3$, $R_4$ and $R_5$ are chosen from among hydrogen, halogens, $C_1$-$C_6$ alkyl, hydroxy, -CHO, -$OR_8$, -COOH, -CN, -$CO_2R_8$, -$CONHR_8$, -$CONR_8R_9$, -$NH_2$, -$NHR_8$, -$N(R_8)_2$, -NH-$CH_2$-$CH_2$-$N(CH_3)_2$, -NH-$CH_2$-$CH_2$-Cl, -$NHCOR_8$, morpholino, nitro, $HSO_3$ groups,

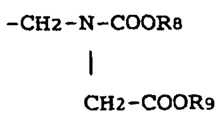 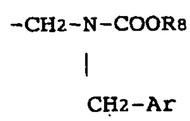

$R_8$ and $R_9$ being chosen from among $C_1$-$C_6$ alkyl groups and ($C_1$-$C_4$) phenyl alkyl groups and Ar is a $C_6$-$C_{14}$ aryl group.